# EUROPEAN PATENT APPLICATION

(11) **EP 2 380 909 A1**
(43) Date of publication of application: **26.10.2011**
(21) Application number: 10075169.2
(22) Date of filing: 26.04.2010
(51) Int. Cl.: C07K 16/28, C12N 15/113

(54) **PTK-7 protein involved in breast cancer**

(71) Applicant: Max-Planck-Gesellschaft zur Förderung der Wissenschaften e.V., 80539 München (DE)
(72) Inventor: Gärtner, Silvia, 82152 Martinsried (DE); Knyazev, Pjiotr Prof., 82131 Stockdorf (DE); Knyazeva, Tatjana Dr., 82152 Martinsried (DE); Gautsch, Renate, 82152 Martinsried (DE); Ullrich, Axel Prof., 80331 München (DE)
(74) Representative: Arth, Hans-Lothar

(57) **Abstract**

The present invention relates to the polypeptide PTK7 and the use thereof as a therapeutic target specifically in the treatment of triple negative (ER-, HER2-, PgR-) breast cancer cells. Further provided are agents which interact with and modulate the oncogenic activity of PTK7 as well as methods for the identification of such agents.

## Description

The present invention relates to the polypeptide PTK7 and the use thereof as a therapeutic target specifically in the treatment of triple negative (ER-, HER2-, PgR-) breast cancer cells and basal-like breast cancer cells. Further provided are agents and compositions, which interact with and modulate the oncogenic activity of PTK7, as well as methods for the identification of such agents.

### Background of the invention

Receptor protein tyrosine kinases transduce extracellular signals across the cell membrane. A subgroup of these kinases lack detectable catalytic tyrosine kinase activity but retain roles in signal transduction. The protein PTK7 also known as CCK4 is a member of this subgroup of tyrosine kinases and may function as a cell adhesion molecule that acts as a regulator of planar cell polarity. This protein is thought to have a function in tumor progression. However, both promoting (Katoh, M., et al., International Journal of molecular medicine, 207, 20, 405-409) and inhibitory (Easty, D., et al., Int. J. Cancer, 1997, 1061-1065) functions in cancer development by PTK7 have been published, depending on the specific type of cancer. Four transcript variants encoding four different isoforms have been found for this gene.

Tumor specific proteins have been identified for a number of cancer types using techniques such as differential screening of cDNAs (Hubert, R. S., et al., Proc. Natl. Acad. Sci. USA 1996, 96, 14523-14528) and the purification of cell-surface proteins that are recognized by tumor-specific antibodies (Catimel, B, et al., J. Biol. Chem. 1996, 271, 25664-25670).

More recently, DNA 'chips' containing up to 10,000 expressed sequence elements have been used to characterize tumor cell gene expression (Dhanasekaran, S. M., et al., Nature 2001, 412, 822-826). However, there are several reasons why the numerous and extensive previous transcriptomic analysis of cancers may not have revealed all, or even most, tumor associated proteins. These include: (i) a lack of correlation between transcript and disease-associated protein levels, particularly common for membrane proteins that often have a long half-life and as such do not have a high mRNA turnover. Therefore, whilst the difference in protein levels between normal and cancerous cells are consistent it is often difficult to associate changes in the mRNA for a given membrane protein with the cancerous state. (ii) Translocation of a protein in the disease state rather than simply differential levels of the transcript, for example of erbB2/HER2-neu, results in much greater plasma-membrane localization in cancer cells than in normal breast cells, the transcription factors oestrogen receptor and STAT3 translocate to the nucleus to exert their tumourigenic effects. (iii) Novel, uncharacterized genes are not highly represented within the 'closed system' of a cDNA array where there are restrictions on the number of expressed sequence elements per chip and the knowledge and availability of DNA clones. It is well established that there is an unreliable relationship between protein expression and mRNA levels (e. g. Gygi, S.P., et al., Mol. Cell Biol. 1999, 19, 1720-1730) as protein expression is subject to strict translational control at several levels. Regulation of the overall activity of the translational apparatus of a cell is expected to affect the translation of essentially all mRNAs.

Indeed, a fraction of specific mRNA is completely repressed. Furthermore individual mRNAs differ greatly in their efficiencies of translation and can be 'weak' or 'strong', thus contributing to the regulation of gene expression. Thus, to identify a protein functional in a specific cellular context or cancerous disease state therefore best requires functional validation of the polypeptide or protein candidate by knockdown or over-expression in a cellular or in vivo assay.

When a protein or polypetide functional in the development of a disease such as cancer has been identified, the said polypeptide or protein may provide a promising screening candidate for the development of new drugs for cancer treatment. Any chemical substances peptides our proteins found to interact with the target protein PTK7 and inhibit the activity of said protein or directly inhibit the expression of the target protein provides a potential drug candidate for the treatment of cancer. Furthermore substances which simply bind to an extracellular oncogene may activate an immune response against the tumor cells expressing the oncogene.

Further the overexpressed oncogene may be used for vaccine development in the treatment of cancer.

Breast cancer is the most frequently diagnosed cancer in women. The implementation of screening programs for the early detection of breast cancer, and the advent of anticancer treatments, such as chemotherapy, radiotherapy and anti-oestrogen therapies, to augment surgical resection have improved the survival of breast cancer patients. However, some breast tumors become refractory to such treatments, as the cancer cells develop resistance to chemotherapy drugs or lose their hormone sensitivity, leading to recurrent or metastatic disease which is often incurable. More recently, attention has focused on the development of immunological therapies such as cancer vaccines and monoclonal antibodies (mAbs), as a means of initiating and targeting a host immune response against tumor cells. Herceptin, a mAb that recognises the erbB2/HER2-neu receptor protein, is used for treatment for metastatic breast cancer in combination with chemotherapy. Herceptin has been shown to prolong the time of disease progression, when compared to patients receiving chemotherapy alone. Herceptin, however, is only effective in treating the 10-20% of patients whose tumors over-express the erbB2/HER2 protein. Thus, important needs exist for new therapeutic agents for the treatment of HER2 negative breast carcinoma.

Another mechanism suggested to play a role in the progression of human breast cancer from hormone dependence to independence is the expression or altered expression of mutant and/or variant forms of the estrogen receptor (ER). It was found that 2 major types of variant ER mRNA had been reported in human breast biopsy samples so far: truncated transcripts and exon-deleted transcripts. In addition a novel type of abnormal ER mRNA was found. Larger-than-wild type ER mRNA RT-PCR products were found in 9.4% of 212 human breast tumors analyzed. Cloning and sequencing of these larger RT-PCR products showed 3 different types: complete duplication of exon 6 in 7.5%; complete duplication of exons 3 and 4 in 1 tumor; and a 69-bp insertion between exons 5 and 6 in 3 tumors. While it is unknown if these novel ER-like mRNAs are stably translated in vivo, any resulting protein would be structurally altered, possibly resulting in altered function.

Antibodies against mutant forms of the estrogen receptor might also have potential to target the hosts immune towards tumor cells, however also here only a very limited number of cancer patients expressing a specific mutated form of the estrogen receptor will benefit from the treatment.

However breast cancer patients lacking the three ligands mentioned above are hard to treat. The object of the present invention is to find alternative treatment particularly helpful for the treatment of triple negative patients.

cDNA encoding PTK-7, also known as CCK-4, was first isolated by Lee et al., (Oncogene 1993, 8, 3403-3410) and later by Mossie et al., (WO 96/37610 A).

Over expression of PTK7 also known as CCK4 was found in 40% (8 out of 20) of tested primary breast cancer cell lines and over-expression surprisingly was predominantly found in triple negative (ER-, HER2-, PgR-) breast cancer cells and basal-like breast cancer cells.

**Triple-negative breast cancer** (TNBC) is a phenotype based on a negative estrogen receptor (ER), HER2 (human epidermal growth factor receptor 2) and progesterone receptor (PgR) as assessed with immunohistochemistry (IHC). As there is an inter-observer variation among pathologists on the estrogen receptor (ER), progesterone receptor (PgR) and HER2 status of breast cancers, the composition of reported triple-negative breast cancer series may vary per study. In addition, ER and HER2 "double-negative" breast cancer can be used as a proxy for triple-negative breast cancer (CA Livasy, G Karaca and R Nanda et al., Phenotypic evaluation of the basal-like subtype of invasive breast carcinoma, Mod. Pathol. 2006, 19, 264-271).

**Basal-like breast cancer** (BLBC) was already described in the late 70-th as a breast cancer subtype originating from the basal layer of mammary epithelium, also known as the myoepithelial cell, but only gained widespread popularity through its molecular definition discovered by Ch. Perou and colleagues in their publication on the molecular portraits of human breast tumors (CM Perou, T Sorlie and MB Eisen et al., Molecular portraits of human breast tumours, Nature 2000, 406 2000, 747-752). The early work from the Stanford group suggested that the basal-like subtype could be characterized IHC by staining for cytokeratin (CK) 5/6.

With their IHC definition of basal-like breast cancer as being ER and HER2 negative, and CK5/6 positive and/or CK14 positive and/or CK17 positive and/or epidermal growth factor receptor (EGFR) positive, with triple (or double) negativity on IHC, and generally reported a concordance of circa 70-80%.

Two definitions of basal-like breast cancer are in use which are also used herein:
1) based on gene expression profiling (considered by many as the "gold standard");
2) based on ER, HER2 and additional basal markers (e.g. CK5/6, CK14, CK17, EGFR) assessed with IHC.

Currently, within the TNBC subtype and the BLBC subtype, neither prognostic nor predictive factors are available to guide treatment decisions. Consequently, overtreatment in the adjuvant setting is common for this subtype, while the treatment given is only moderately effective, due to the lack of predictive factors, i.e. targeted therapy. Now the challenge is to identify "druggable" molecular changes driving tumourigenesis in TNBC as well as in BLBC. Additionally a polyclonal antibodies raised against the extracellular domain of PTK7 surprisingly inhibited PTK7 activity and was thereby able to reduce the ability of Hs578T (wt) breast cancer cells to outgrow (form colonies) in matrigel. Consequently the present invention provides a new method to treat ER, HER-2 and PgR triple negative breast cancer patients especially to prevent breast cancer angiogenesis, invasion and metastasis in triple negative (ER, HER-2, PgR) breast cancer cells. The present invention also provides a new method to treat ER and HER-2 negative and CK5/6 and/or CK14 and/or CK17 and/or EGFR positive basal-like breast cancer patients especially to prevent breast cancer angiogenesis, invasion and metastasis in basal-like breast cancer cells. Moreover the present invention demonstrates for the first time that inhibition of PTK7 by binding of an inhibitor or an antibody to the extracellular domain of the polypeptide can reduce breast cancer cell invasion and metastatic outgrowth. Furthermore the present invention demonstrates that PTK7 (also named as CCK4) has oncogenic (transforming) function in the two breast cancer subtypes basal-like breast cancer and triple negative breast cancer. It is shown that PTK7 (CCK4) is a key signaling pathway for metastasis development via control of "polar cell polarity" (PCP), cancer cell orientation or cancer cell navigation in the space of tissues, substrate attachments and motility. PTK7/CCK4 signaling pathway give an individual cancer cell self guidance towards one (unic) and significant direction. PTK7 is an control of non-canonical Wnt-signalling pathway for PCP, attachment and motility. All these functions of PTK7 are used by cancer cells for metastasis development.

### Description of the invention

The present invention is based on the finding that PTK7 represents a novel therapeutic target for the treatment and/or prophylaxis of triple negative (ER, HER2, PgR) breast cancer and basal-like breast cancer (ER and HER2 negative and at least one of CK5/6, CK14, CK17 and EGFR positive) and that a PTK7 specific polyclonal antibody was able to inhibit colony outgrowth of the triple negative breast cancer cell line Hs5787 in matrigel.

Endothelial cells cultured on matrigel create intricate colony outgrowth leading to spiderweb-like networks on matrigel coated surfaces but not on plastic surfaces. Such networks are highly suggestive of the microvascular capillary systems that suffuse living tissues with blood. Therefore the assay measures the angiogenic and invasive capacity of tumor cells. Inhibition of this activity in tumor cells thereby provides a method for cancer treatment especially for the inhibition of cancer metastasis.

Accordingly, the invention provides a method for the treatment and/or prophylaxis of triple negative (ER-, HER2-, PgR-) breast cancer and carcinoma and method for the treatment and/or prophylaxis of basal-like breast cancer, especially for the inhibition of angiogenesis, invasion and metastasis of triple negative (ER-, HER2-, PgR-) breast carcinoma cells and basal-like breast cancer cells, preferentially by administering a therapeutically effective amount of an agent which interacts with or modulates the expression or activity of a PTK7 polypeptide.

Thus, the present invention is directed to a pharmaceutical composition for the treatment and prophylaxis of angiogenesis, invasion and metastatic outgrowth of the breast cancer subtypes triple negative breast cancer and basal-like breast cancer comprising an agent which interacts with or modulates the expression or activity of a PTK7 polypeptide. This agent contained in the pharmaceutical composition is able to downregulate the expression of the PTK7 polypeptide and / or is able to decrease the activity of the PTK7 polypeptide. Preferably, such an agent is an antibody against PTK7 or a functionally active fragment, derivative or analogue of that antibody or is a taxane as disclosed below and preferably paclitaxel or is a combination of a DNA damaging agent and a PARP inhibitor.

As used herein the triple negative breast cancer is characterized by the three negative receptors ER, PgR and HER2 and the basal-like breast cancer is characterized by the two negative receptors ER and HER2 and at least one positive receptor of the group consisting of CK5/6, CK14, CK17 and EGFR.

As used herein the term "negative" refers to the fact that ER, HER2 and PgR are expressed in breast cancer tissues.
As used herein the term "positive" refers to the fact that ER, HER2 and PgR are not expressed in breast cancer tissues or are expressed very weakly.

A PTK7 polypeptide includes a polypeptide which: (a) comprises or consists of the amino acid sequence of SEQ ID NO: 1 or SEQ ID NO: 2 or SEQ ID NO: 3 or SEQ ID NO: 4 and especially SEQ ID NO: 1. The amino acid sequences SEQ ID NO: 1, SEQ ID NO: 2, SEQ ID NO: 3 and SEQ ID NO: 4 are four splice variants of PTK7. The term PTK7 polypeptide preferably refers to SEQ ID NO: 1 and parts thereof.

The term "polypeptides" includes proteins, peptides, polypeptides and oligopeptides. These terms are used interchangeably unless otherwise specified.

Agents of use in the methods of the invention include without limitation, agents that are capable of interacting with (e. g. binding to, or recognizing) a PTK7 polypeptide or inhibiting the expression of a nucleic acid molecule encoding a PTK polypeptide or a PTK7 polypeptide, or are capable of modulating the interaction or activity of a PTK7 polypeptide.

The skilled person will therefore appreciate that the methods of the invention include direct targeting and inhibition of the PTK7 polypeptide, preferentially by using antibodies binding directly to the extracellular domain of the peptide but also by other agents which can bind to the peptide such as carbohydrates, lipids, proteins, polypeptides, peptides, peptidomimetics, small molecules and other drugs, as well as indirectly affecting the expression of the polypeptide by targeting the corresponding nucleic acid.

Thus, the invention also provides the use of an agent wherein this agent could also be a combination of more than one active ingredients or drugs, which modulates the activity of a PTK7 polypeptide in the manufacture of a medicament for the treatment and/or prophylaxis of triple negative (ER, HER-2, PgR) breast cancer cells. Moreover the invention also provides the use of an agent wherein this agent could also be a combination of more than one active ingredients or drugs, which modulates the activity of a PTK7 polypeptide in the manufacture of a medicament for the treatment and/or prophylaxis of basal-like (ER and HER2 negative and at least one of CK5/6, CK14, CK17 and EGFR positive) breast cancer cells.

Most preferably, the agent for use in the prophylaxis and/or treatment of triple negative breast cancer cells or basal-like breast cancer cells is an antibody which interacts with (i. e. binds to or recognizes) or modulates the activity of a PTK7 polypeptide by specifically binding to the extracellular domain of the polypeptide. Specifically recognizing or binding specifically means that the antibodies have a greater affinity for PTK polypeptides than for other polypeptides. Such antibodies may inhibit PTK7 activity due to the direct interaction with the polypeptide and thereby inhibit the formation of metastasis and spread of the breast cancer but also may enhance the body's immune response against the breast cancer cells expressing PTK7.

In particular, an antibody which specifically interacts with a PTK polypeptide may be used to mediate antibody dependent cell cytotoxicity (ADCC) and complement dependent cytotoxicity (CDC).

Accordingly, an antibody which specifically recognizes a PTK polypeptide shall be used in the manufacture of a medicament for the treatment of triple negative breast carcinoma or breast cancer cells and/or the fight against metastasis of triple negative (ER, HER-2, PgR) breast carcinomas or breast cancer cells. Also accordingly, an antibody which specifically recognizes a PTK polypeptide shall be used in the manufacture of a medicament for the treatment of basal-like breast cancer cells and/or the fight against metastasis of basal-like breast cancer cells.

Thus, the present invention relates to the use of an agent which interacts with or modulates the expression or activity of a PTK7 polypeptide for the manufacture of a pharmaceutical composition for the treatment and prophylaxis of angiogenesis, invasion and metastatic outgrowth of triple negative breast cancer or of basal-like breast cancer. Preferably, the agent or the antibody is able to downregulate the expression of the PTK7 polypeptide and / or wherein the agent or antibody is able to decrease the activity of the PTK7 polypeptide.

Surprisingly, it was found that triple-negative breast cancer and basal-like breast cancer are highly sensitive to a combination of a DNA damaging agent, such as a platinum compounds, *cis*-diamminedichloroplatinum (II) (cisplatin), Carboplatin (JM-8, paraplatin), *cis*-dichloro-*trans*-dihydroxy*-cis-bis*(isopropylamine)platinum(IV) (Iproplatin, CHIP), (aquo)sulfato(1,1-bis-aminomethylcyclohexane)platinum(II) (TNO-6), oxaliplatin, Tetraplatin (ormaplatin), *cis-*amminedichloro(cyclohexylamine)platinum(II) (JM118), *cis-*amminedichloro(cyclohexylamine)-*trans*-dihydroxoplatinum(IV) (JM149), bis-acetato-*cis-*amminedichloro(cyclohexylamine)platinum(IV) (JM216), *trans-*amminedichloro(cyclohexylamine)dihydroxoplatinum(IV) (JM335), Transplatin, *cis,trans,cis-*Pt(NH₃)(C₆H₁₁NH₂)(OOCC₃H₇)₂Cl₂, Nedaplatin (254S), malanato-1,2-diaminocyclohexaneplatinum(II), 5-sulfosalicylato-*trans*-(1,2 diaminocyclohexane)platinum(II) (SSP), poly-[(*trans*-1,2-diaminocyclohexane)platinum]-carboxyamylose (POLY-PLAT), and 4-hydroxy-sulfonylphenylacetato(*trans*-1,2-diaminocyclohexane) platinum(II) (SAP) and a PARP inhibitors such as BSI 201 (4-iodo-3-nitrobenzamide), Olaparib (was AZD-2281), ABT-888, AG014699, CEP 9722, MK 4827, KU-0059436 (AZD2281) and LT-673.

Moreover it was surprisingly found that triple-negative breast cancer and basal-like breast cancer can effectively treated with taxanes such as paclitaxel, Taboo^{®}, taxotere, baccatin, 7-xylosyl-10-deacetyltaxol, cephalomannine, 10-deacetyl-7-epitaxol, 7 epitaxol, 10-deacetylcephaolmannine, 7-deoxy-docetaxol, 7,8-cyclopropataxanes, N-substituted 2-azetidones, 6,7-epoxypaclitaxel, 6,7-modified paclitaxel, 10-desacetoxytaxol, 10-deacetyltaxol (from 10-deacetylbaccatin III), phosphonooxy and carbonate derivatives of taxol, taxol 2',7-disodium-1,2-benzenedicarboxylate, 10-desacetoxy-11,12-dihydrotaxol-10,12(18)-diene derivatives, 10-desacetoxytaxol, Protaxol (2'-and/or 7-O-ester derivatives), 2'-and/or 7-O-carbonate derivatives of paclitaxel, fluoro taxols, 9-deoxotaxane, 13-acetyl-9-deoxobaccatine III, 9-deoxotaxol, 7-deoxy-9-deoxotaxol, 10-desacetoxy-7-deoxy-9-deoxotaxol, sulfonated 2'-acryloyltaxol, sulfonated 2'-O-acyl acid taxol derivatives, succinyltaxol, 2'-gamma-aminobutyryltaxol, 2'-acetyl taxol, 7-acetyl taxol, 7-glycine carbamate taxol, 2'-OH-7-PEG(5000) carbamate taxol, 2'-benzoyl derivatives of paclitaxel, 2',7-dibenzoyl taxol, 2'-acetyltaxol; 2',7-diacetyltaxol; 2'-succinyltaxol; 2'-(beta-alanyl)-taxol, ethylene glycol derivatives of 2'- succinyltaxol, 2'-glutaryltaxol, 2'-(N,N-dimethylglycyl) taxol, 2'-(2-(N,N-dimethylamino)propionyl)taxol, 2'-orthocarboxybenzoyl taxol, 2'-aliphatic carboxylic acid derivatives of paclitaxel, 2'-(N,N-diethylaminopropionyl)taxol, 7-(N,N-dimethylglycyl)taxol, 2',7-di-(N,N-dimethylglycyl)taxol, 7-(N,N-diethylaminopropionyl)taxol, 2',7-di(N,N-diethylaminopropionyl)taxol, 2'-(L-glycyl)taxol, 7-(L-glycyl)taxol, 2',7-di(L-glycyl)taxol, 2'-(L-alanyl)taxol, 7-(L-alanyl)taxol, 2',7-di(L-alanyl)taxol, 2'-(L-leucyl)taxol, 7-(L-leucyl)taxol, 2',7-di(L-leucyl)taxol, 2'-(L-isoleucyl)taxol, 7-(L-isoleucyl)taxol, 2',7-di(L-isoleucyl)taxol, 2'-(L-valyl)taxol, 7-(L-valyl)taxol, 2'7-di(L-valyl)taxol, 2'-(L-phenylalanyl)taxol, 7-(L-phenylalanyl)taxol, 2',7-di(L-phenylalanyl)taxol, 2'-(L-prolyl)taxol, 7-(L-prolyl)taxol, 2',7-di(L-prolyl)taxol, 2'-(L-lysyl)taxol, 7-(L-lysyl)taxol, 2',7-di(L-lysyl)taxol, 2'-(L-glutamyl)taxol, 7-(L-glutamyl)taxol, 2',7-di(L-glutamyl)taxol, 2'-(L-arginyl)taxol, 7-(L-arginyl)taxol, 2',7-di(L-arginyl)taxol, (N-debenzoyl-N-tert-(butoxycaronyl)-10-deacetyltaxol, baccatin III, cephalomannine, 10-deacetylbaccatin 111, brevifoliol, yunantaxusin, taxusin, 14-beta-hydroxy-10-deacetybaccatin III, debenzoyl-2-acyl paclitaxel derivatives, benzoate paclitaxel derivatives, phosphonooxy paclitaxel derivatives, carbonate paclitaxel derivatives, sulfonated 2'-acryloyltaxol; sulfonated 2'-O-acyl acid paclitaxel derivatives, 18-site-substituted paclitaxel derivatives, chlorinated paclitaxel analogues, C4 methoxy ether paclitaxel derivatives, sulfonamide taxane derivatives, brominated paclitaxel analogues, Girard taxane derivatives, nitrophenyl paclitaxel, 10-deacetylated substituted paclitaxel derivatives, 14-beta-hydroxy-10-deacetylbaccatin III taxane derivatives, C7 taxane derivatives, 2-debenzoyl-2-acyl taxane derivatives, 2-debenzoyl paclitaxel derivatives, 2-acyl paclitaxel derivatives, 10-deacetyl taxol A, 10-deacetyl taxol B, 2-aroyl-4-acyl paclitaxel analogues, orthro-ester paclitaxel analogues.

A therapeutic antibody as disclosed in the present invention may be used alone or may be conjugated to a therapeutic moiety such as a cytotoxic agent, a radionuclide or drug moiety to modify a given biological response.

The therapeutic agent can be a chemical therapeutic agent but may also be a drug moiety which may be a protein or polypeptide possessing a desired biological activity. Such moieties may include, for example and without limitation, a toxin such as abrin, ricin A, pseudomonas exotoxin, or diphtheria toxin, a protein such as tumour necrosis factor, alpha-interferon, β-interferon, nerve growth factor, platelet derived growth factor or tissue plasminogen activator, a thrombotic agent or an anti-angiogenic agent, e. g. paclitaxel, angiostatin or endostatin, or a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G- CSF), nerve growth factor (NGF) or other growth factors.

Therapeutic agents also include any agent that is detrimental to (e. g. kills) cells, or further stopps proliferation and invesion of cancer cells. Examples include alkylating agents (e. g. mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), cytoskeletal disruptors, inhibitors of topoisomerase II, anthracyclines (e. g. daunorubicin (formerly daunomycin)), nucleotide analogs and precursor analogs, platinum-based agents, retinoids, vinca alkaloids and derivatives, antimetabolites (e. g. methotrexate, 6-mercaptopurine, 6-thioguanine, cytarabine, 5-fluorouracil decarbazine), and preferentially the breast cancer therapeutic agents paclitaxel, trastuzumab, capecitabine, letrozole, exemestane, anastrozole, lapatinib, doxorubicin, cyclophosphamide, fluorouracil, docetaxel, methotrexate, gemcitabine, epirubicin, vinorelbine, fulvestrant, toremifene, paclitaxel protein-bound, testolactone, bevacizumab. Further especially preferred cytotoxins or cytotoxic agents include cytochalasin B, gramicidin D, ethidium bromide, emetine, mitomycin, etoposide, tenoposide, vincristine, vinblastine, colchicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, but are not limited to, antibiotics (e. g. dactinomycin (formerly actinomycin), bleomycin, mithramycin, anthramycin (AMC), calicheamicins or duocarmycins), and anti-mitotic agents (e. g. vincristine and vinblastine).

Other therapeutic moieties may include radionuclides such as ¹¹¹In and ⁹⁰Y, ¹⁷⁷Lu, Bismuth ²¹³, Californium²⁵², Iridium¹⁹², Tunsten¹⁵⁸ and Rhenium¹⁸³ or drugs such as but not limited to, alkylphosphocholines, topoisomerase I inhibitors, taxoids and suramins.

The antibodies disclosed herein can be conjugated with the therapeutic agents mentioned above. The antibody therapeutic agent conjugates may be covalently attached to each other as for example disclosed in the patent application WO 1986/001720 A. Such techniques are well known to the art.

The antibodies for use in the invention include analogues and derivatives that are modified, for example but without limitation, by the covalent attachment of any type of molecule as specified above.

Preferably, said attachment does not impair immunospecific binding. In one aspect, an antibody can be conjugated to a second antibody to form an antibody heteroconjugate (see for example US 4,676, 980).

In other embodiments, the invention provides the therapeutic use of fusion proteins of the antibodies (or functionally active fragments thereof), for example but without limitation, where the antibody or fragment thereof is fused via a covalent bond (e. g. a peptide bond), at optionally the N-terminus or the C-terminus, to an amino acid sequence of another protein (or portion thereof; preferably at least a 10, 20 or 50 amino acid portion of said another protein). Preferably the antibody, or fragment thereof, is linked to the other protein at the N-terminus of the constant domain of the antibody.

Where the fusion protein is an antibody fragment linked to an effecter molecule, this may be prepared by standard chemical or recombinant DNA procedures. For conjugation reagents, reagents as disclosed in U.S. Pat. No. 5,053,520 may be used. In this method, heterobifunctional linkers having extended alkyl, cycloalkyl, alkylcycloalkyl and aromatic portions are used to couple an antibody to an enzyme. Further preferred linkers are homo-bifunctional, bis-maleimidopolyalkylene glycol linkers such as described in U.S. Pat. No. 4,810,638. Furthermore, polyalkyleneglycol linkers such as described in US20060246523 are preferred objects of the invention.

Particular chemical procedures include, for example, those described in WO 93/62331 A, WO 92/22583 A, WO 90/195 A and WO 89/1476 A. Alternatively, where the effecter or reporter molecule is a protein or polypeptide the linkage may be achieved using recombinant DNA procedures, for example as described in WO 86/01533 A and EP 0392745 A.

Further antibodies can be attached to poly(ethyleneglycol) (PEG) moieties.

PTK polypeptides or cells expressing said polypeptides can be used to produce antibodies, e. g. which specifically recognize said PTK7 polypeptides. Antibodies generated against a PTK7 polypeptide may be obtained by administering the polypeptides to an animal, preferably a non-human animal, using well-known and routine protocols.

Anti-PTK7 antibodies include functionally active fragments, derivatives or analogues and may be, but are not limited to, polyclonal, monoclonal, bispecific, humanized or chimeric antibodies, single chain antibodies, Fab fragments and F (ab') fragments, fragments produced by a Fab expression library, anti-idiotypic (anti-Id) antibodies, and epitope-binding fragments of any of the above. Humanized antibodies are antibody molecules from non-human species having one or more complementarity determining regions (CDRs) from the non-human species and a framework region from a human immunoglobulin molecule (see, e. g. US 5,585, 089). Antibodies include immunoglobulin molecules and immunologically active portions of immunoglobulin molecules, i. e. molecules that contain an antigen binding site that specifically binds an antigen. The immunoglobulin molecules of the invention can be of any class (e. g. IgG, IgE, IgM, IgD and IgA) or subclass of immunoglobulin molecule.

Monoclonal antibodies may be prepared by any method known in the art such the hybridoma technique, the trioma technique, the human B-cell hybridoma technique and the EBV-hybridoma technique.

Chimeric antibodies are those antibodies encoded by immunoglobulin genes that have been genetically engineered so that the light and heavy chain genes are composed of immunoglobulin gene segments belonging to different species. These chimeric antibodies are likely to be less antigenic. Bispecific antibodies may be made by methods known in the art.

The antibodies for use in the invention may be generated using single lymphocyte antibody methods based on the molecular cloning and expression of immunoglobulin variable region cDNAs generated from single lymphocytes that were selected for the production of specific antibodies such as described in WO 92/02551 A.

The antibodies for use in the present invention can also be generated using various phage display methods known in the art and include those disclosed in WO 90/02809 A, WO 91/10737 A, WO 92/01047 A, WO 92/18619 A, WO 93/11236 A, WO 95/15982 A, WO 95/20401 A, US 5,698, 426, US 5,223, 409, US 5,403, 484, US 5,580,717, US 5,427,908, US 5,750,753, US 5,821,047, US 5,571,698, US 5,427,908, US 5,516,637, US 5,780,225, US 5,658,727, US 5,733, 743 and US 5,969,108.

Techniques for the production of single chain antibodies, such as those described in US 4,946,778 can also be adapted to produce single chain antibodies to PTK7 polypeptides.

Also, transgenic mice, or other organisms, including other mammals, may be used to express humanized antibodies.

### Pharmaceutical compositions

Thus another aspect of the present invention relates to pharmaceutical composition containing the agent or the anti-PTK7 antibody including functionally active fragments, derivatives or analogues of said anti-PTK antibody which include polyclonal, monoclonal, bispecific, humanized or chimeric antibodies, single chain antibodies, Fab fragments and F (ab') fragments, fragments produced by a Fab expression library, anti-idiotypic (anti-Id) antibodies, and epitope-binding fragments of any one of the above compounds together with at leaqst one pharmaceutically acceptable solvent, diluent, carrier, excipient and/or adjuvant. The term "agent" also covers combinations of two or more pharmaceutically acitve agents. Thus, the pharmaceutical compositions of the present invention contain the antibodies disclosed herein or at least one therapeutic agent other than an antibody or at least one conjugate of an antibody with a therapeutic agent or a taxane or a combination of combination of a DNA damaging agent and a PARP inhibitor.

The preferred pharmaceutical compositions are in administratable form which is suitable for oral application. These administratable forms, for example, include pills, tablets, film tablets, coated tablets, capsules, powders and deposits. Forms other than oral administratable are also possible. The pharmaceutical compositions containing said agent or antibody or antibody fragment may be administered by any appropriate means, including but not limited to inhalation, injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrically, intracutaneously, intravaginally, intravasally, intranasally, intrabuccally, percutaneously, sublingually, or any other means available within the pharmaceutical arts.

Within the disclosed methods the pharmaceutical compositions of the present invention, containing at least one agent or antibody or antibody fragment as an active ingredient will typically be administered in admixture with suitable carrier materials suitably selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active ingredient may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may consist of inventive compositions from about 5 to about 95 percent.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby to solidify.

Also included are solid form preparations which are intended to be converted, shortly before use, to liquid form preparations for either oral or parenteral administration. Such liquid forms include solutions, suspensions and emulsions.

The agent or antibody or antibody fragment may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refer to the active ingredients dispersed or solubilized in a hydrophilic semi-solid matrix.

Powders for constitution refer to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight, and most preferably from about 40 to 50% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and d'I-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.01 to 10% by weight of the composition, preferably from about 0.05 to 6% by weight, more preferably from about 0.1 to about 4% by weight of the composition, and most preferably from about 0.1 to about 1%.

A suitable composition comprising at least one agent or antibody or antibody fragment may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

Consequently, the present invention also relates to the use of the agent as defined herein or antibody or antibody fragment, antibody derivative or antibody analogue as defined herein which interacts with or modulates the expression or activity of a PTK7 polypeptide for the manufacture of a pharmaceutical composition for the treatment and prophylaxis of angiogenesis, invasion and metastatic outgrowth of triple negative breast cancer (ER, HER-2, PgR all negative) or of basal-like breast cancer (ER and HER2 negative and at least one of CK5/6, CK14, CK17 and EGFR positive). All the pharmaceutical compositions and the uses of the agents which interact with or modulate the expression or activity of a PTK7 polypeptide can also be used for the treatment of basal-like breast cancer wherein ER and HER2 are negative and wherein at least one of CK5/6, CK14, CK17 and EGFR is positive. Consequently, all the statements, embodiments, pharmaceutical compositions, agents and examples disclosed in regard to triple negative breast cancer (ER, HER-2, PgR all negative) do also apply to basal-like breast cancer wherein ER and HER2 are negative and wherein at least one of CK5/6, CK14, CK17 and EGFR is positive. Thus all sentences in the present application which cite and refer to triple negative breast cancer (ER, HER-2, PgR) can also be cited and refer also to basal-like breast cancer wherein ER and HER2 are negative and wherein at least one of CK5/6, CK14, CK17 and EGFR is positive. Consequently in all sentences and examples disclosed in the present application, the term "triple negative breast cancer (ER, HER-2, PgR)" or the term "triple negative breast cancer" can be replaced by the term "basal-like breast cancer wherein ER and HER2 are negative and wherein at least one of CK5/6, CK14, CK17 and EGFR is positive".

It is mentioned again that the antibody, the fragment, analogue or derivative thereof may be used in combination with said agent which might also be another antibody or is conjugated to said agent. Conjugation could mean a covalent bond, hydrogen brigdes, adhesion, ionic bond, or a binding due to polar or Van der Waals forces.

The term "interact" or "modulate" preferably refer to a downregulation of the expression of the PTK7 polypeptide and / or to a decrease of the activity of the PTK7 polypeptide.

A further aspect of the present invention is directed to a method of screening for an agent of triple negative (ER, HER2, PgR) breast carcinomas or breast cancer cells or basal-like breast cancer cells for the treatment and prophylaxis of angiogenesis, invasion and metastasis of triple negative (ER, HER2, PgR) breast carcinoma cells, wherein said agent interacts with or modulates the expression or activity of the PTK7 polypeptide, said method comprising: (a) contacting the PTK7 polypeptide with a candidate agent; and (b) determining whether or not the candidate agent interacts with said PTK7 polypeptide.

Said method preferably comprises the further steps c) and d):
c) comparing the expression or activity of the PTK7 polypeptide in the presence of the candidate agent with the expression or activity of the PTK7 polypeptide in the absence of the candidate agent or in the presence of a control agent; and d) determining whether the candidate agent causes the expression or activity of the PTK7 polypeptide to change.

This method is used for the identification of agents which are able to interact with or modulate the expression or activity of a PTK7 polypeptide and preferably which are able to decrease or downregulate the expression of the PTK7 polypeptide and / or to a decrease or reduce or inhibit of the activity of the PTK7 polypeptide.

### Description of the figures:

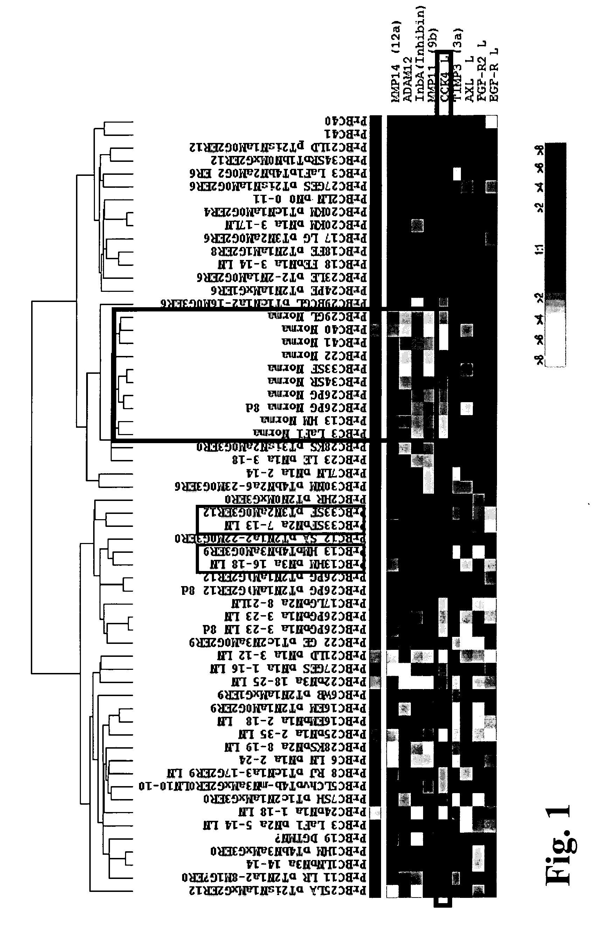

- Figure 1: is a classification of the normal breast tissues and primary breast cancer (BC) tissues by gene expression profiling. The expression of 8 genes which comprise known kinases and others cancer related genes which were identified in aggressive breast cancer cell lines was investigated by an unsupervised cDNA array analysis. The level of gene expression relative to normal breast tissues (mix of two) is shown by the colour and shade designated in the key at the bottom of the cluster. Each colour shade encompasses all of the values in the range spanned by the numbers beneath the scale. The blue box indicates normal tissues. The pink boxes indicate pairs of BC with a gene expression which have a slightly elevated gene expression of PTK7/CCK4. The light box marks the PTK7/CCK4 position. Gene expression was measured by cDNA array hybridization of RNA (duplicate preparations) from each of the indicated primary tumours, normal tissues and lymph nodes, as described in "Materials and Methods."

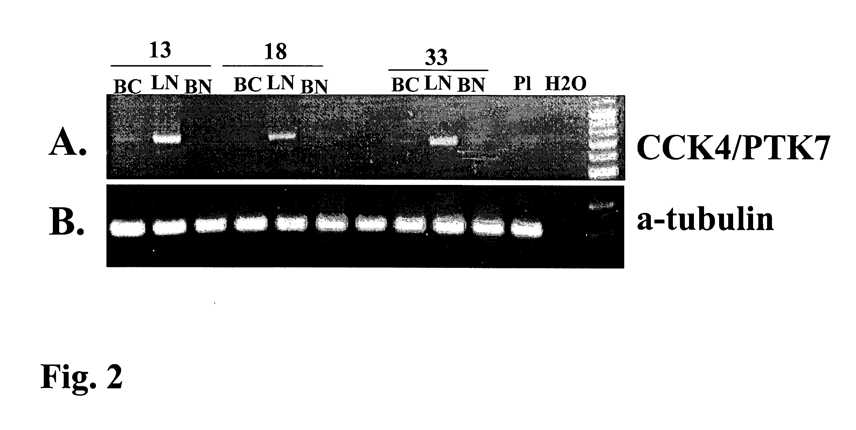

- Figure 2: is a PCR analysis of the expression of PTK7 primary breast cancer (BC), lymph nodes (LN), and normal control breast tissue (BN) from three representative patients (13, 18, 33). A: PTK7/CCK4 product, B: alpha-tubulin control. PI: placenta tissue, H₂O: negative control of PCR.

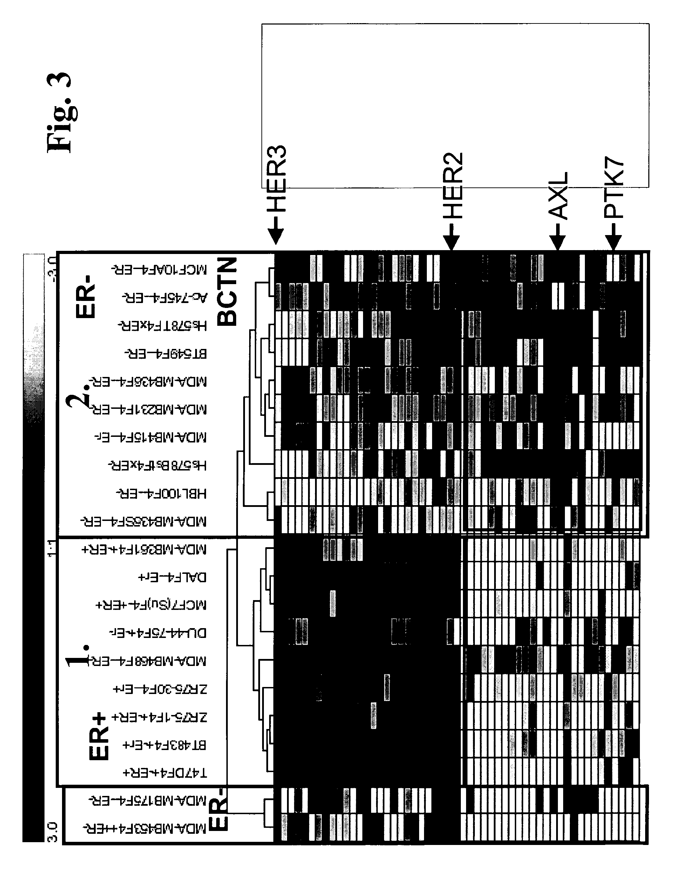

- Figure 3: illustrates the distribution of the expression of PTK7 in breast cancer cell lines grouped by the estrogene receptor status. 1. ER-, HER2-, HER3-positive cell lines, red box, 2. ER-, HER2-, HER3-negative (triple negative, TNBC) yellow boxes. The colors are z-score normalized to depict relative values within rows. They cannot be used to compare values between rows. On top are the names of the cell lines together with the indication of the array tree. On the left are the names of the genes; the red arrows indicate the significant genes.

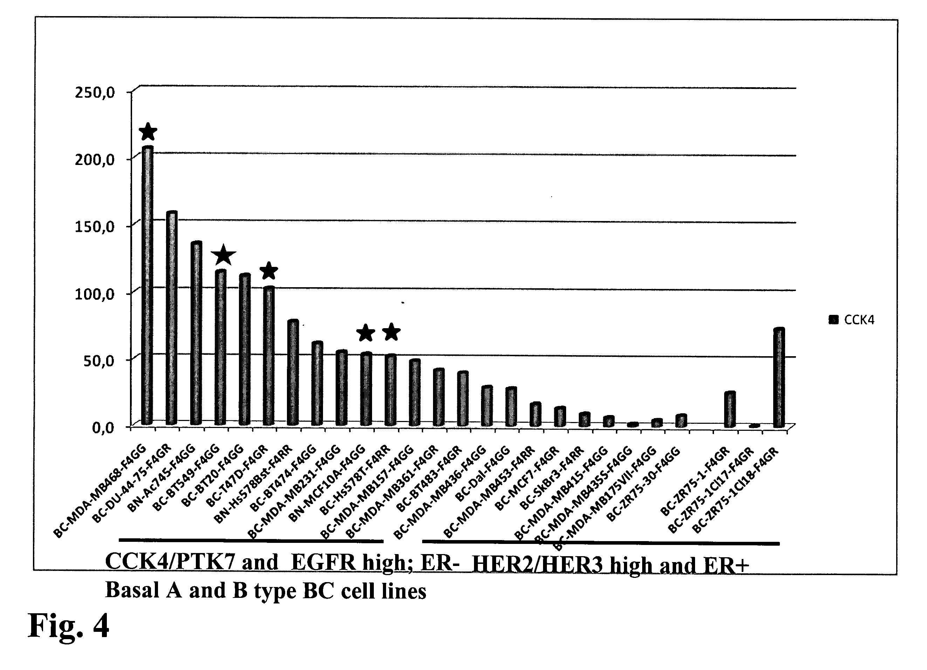

- Figure 4: illustrates the classification of the breast cancer cell lines by expression ot the PTK7/CCK4, estrogen receptor and Her2-/Her3-oncogenes. The red stars indicate genetically modified cell lines which stably express dn-CCK4 mutants or si-RNA-CCK4/PTK7. Cell lines on the side of the red bar highly expressed PTK7/CCK4 and EGFR while there were mainly negative for expression of ER and Her2/Her3. Cell lines on the side of the blue bar were positive for ER and Her2/Her3 expression and were mainly negative of PTK7/CCK4-expression.

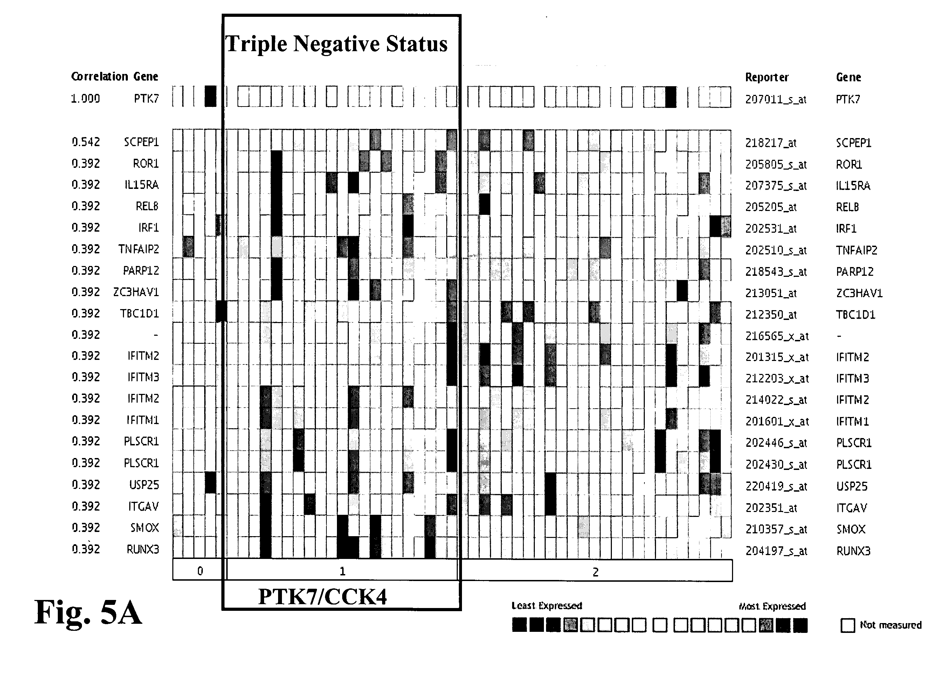

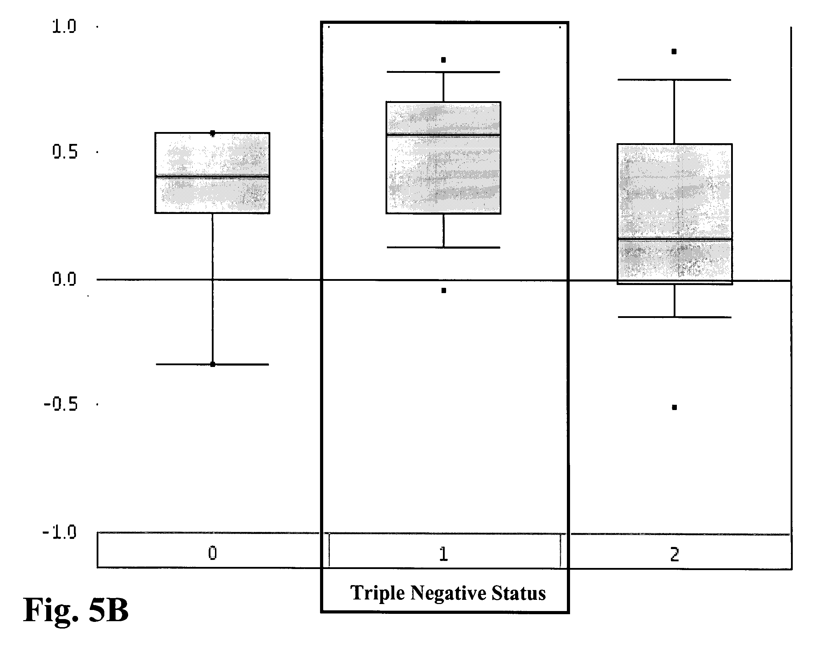

- Figure 5: shows the expression of genes which are co-expressed with PTK7 in 51 breast cancer cell lines (Neve, Oncomine), which were grouped by triple negative status. The colors relate to expression units which are z-normalized to depict relative values within rows. They cannot be used to compare values between rows. A: Hit map: 0. No value (5), 1. ErbB2(HerR2)/ER/PR Negative (21), 2. Other Biomarker Status (25). B: Breast cancer cell line statistics: 0. No value (5), 1. ErbB2 (Her2)/ER/PR Negative (21), 2. Other biomarker status (25).

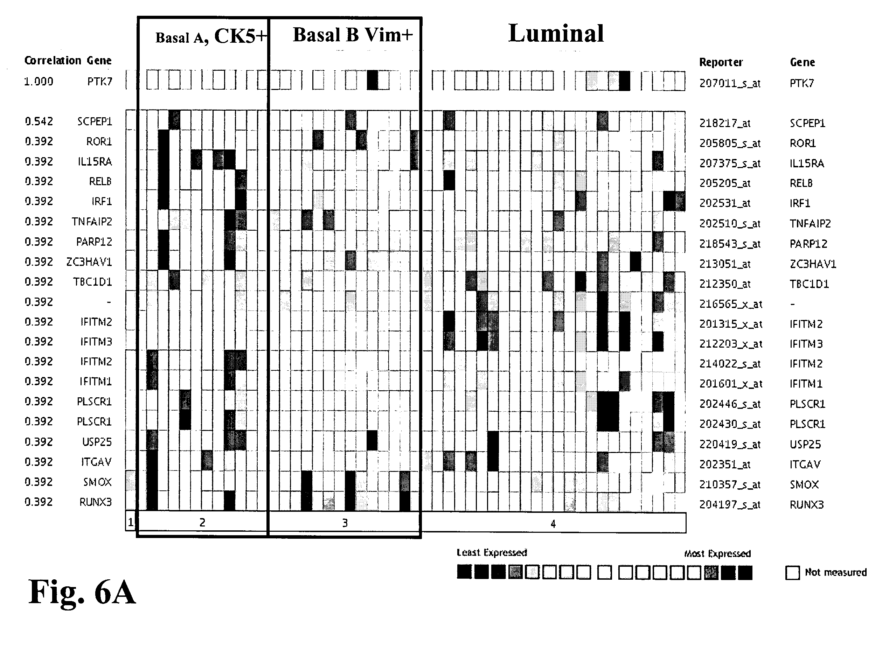

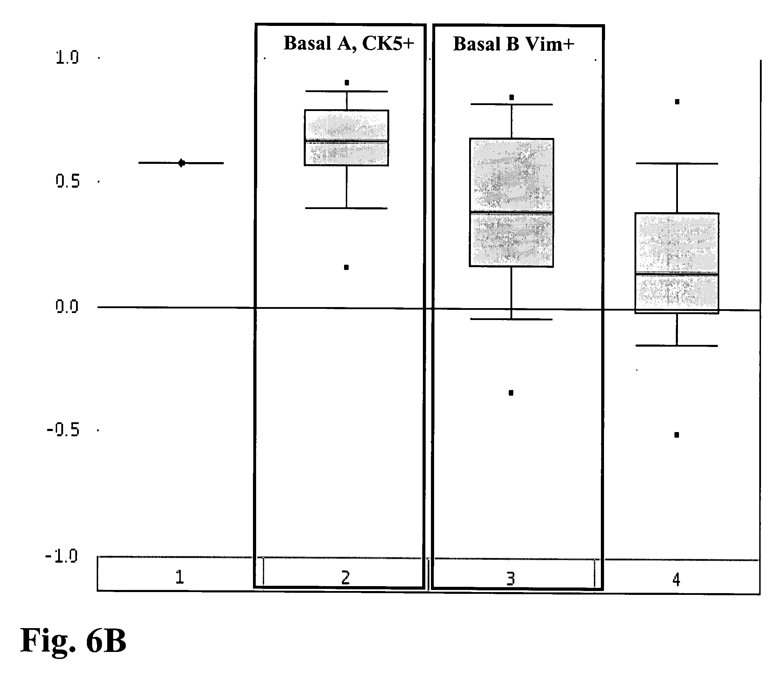

- Figure 6: shows the expression of genes which are co-expressed with PTK7 in 51 breast cancer cell lines (Neve, Oncomine). The colors relate to expression units which are z-normalized to depict relative values within rows. They cannot be used to compare values between rows. A: Hit map: 1. NA (1), 2. Basal A [KRT5-, KRT14-positive] (12), 3. Basal B [VIM-positive] (14), 4. Luminal (24). B: Breast cancer cell line statistics: 1. NA (1), 2. Basal A [KRT5-, KRT14-positive] (12), 3. Basal B [VIM-positive] (14), 4. Luminal (24).

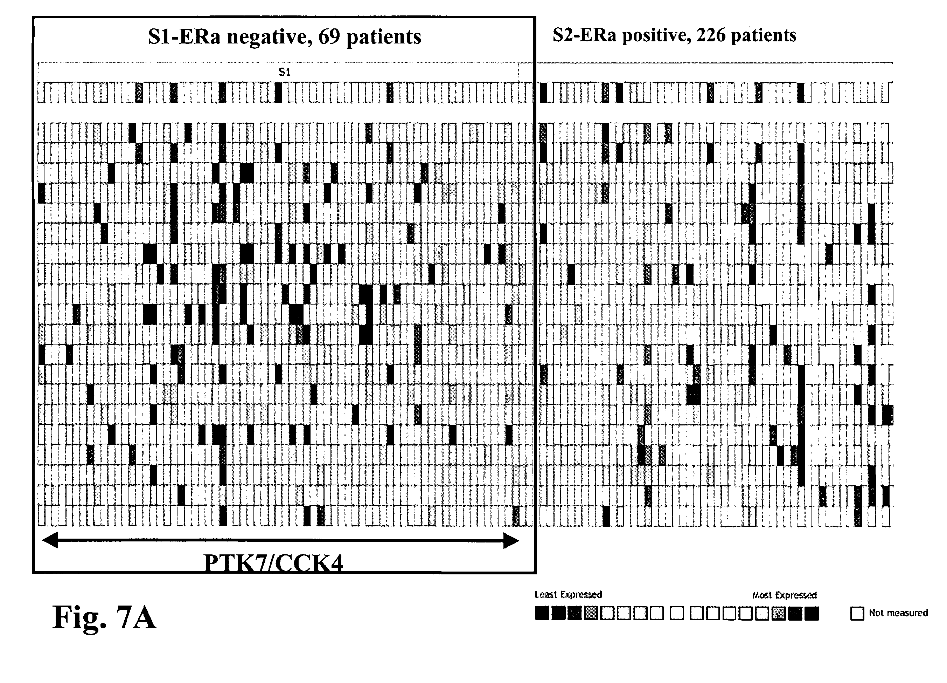

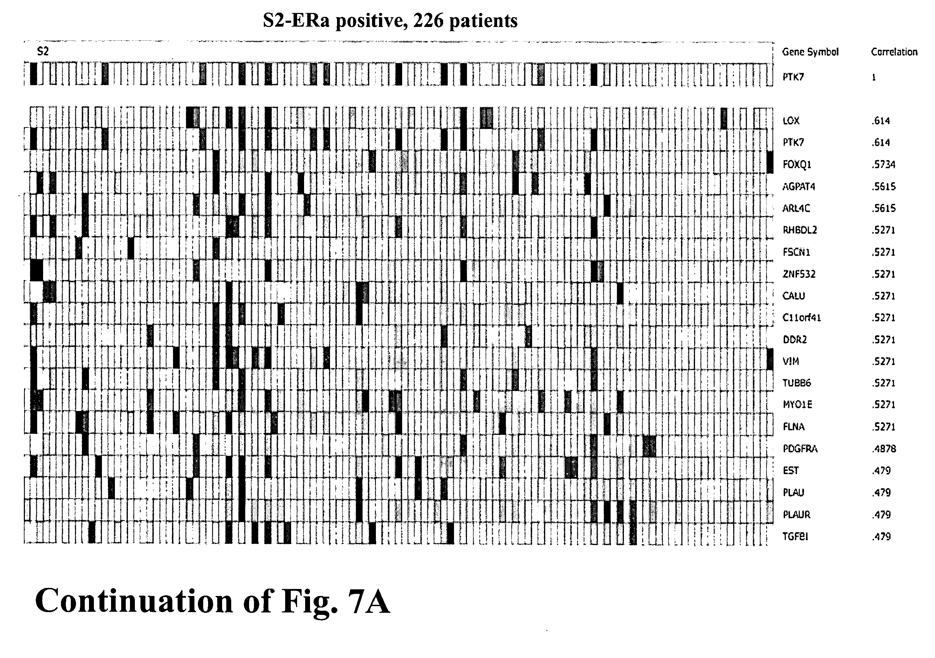

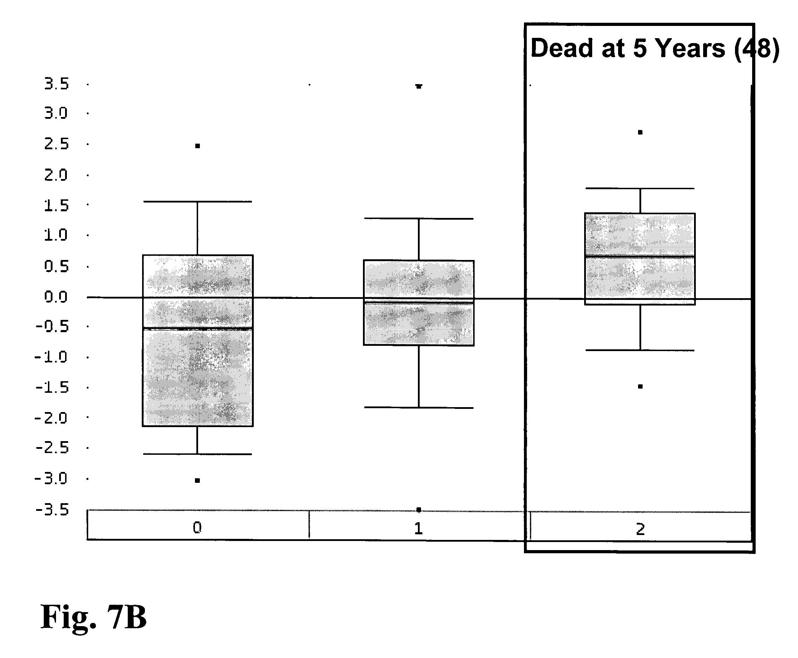

- Figure 7: shows the expression of genes co-expressed with PTK7 in primary breast carcinomas (van de Vijver, Oncomine) which were grouped by ER and survival status. The colors relate to expression units which are z-normalized to depict relative values within rows. They cannot be used to compare values between rows. A. Hit-map: PTK7 expression in van de Vijver's study of breast cancer grouped by a survival status of 5 years; 0: no value (15), 1: alive after 5 years (232), 2: dead after 5 years (48).

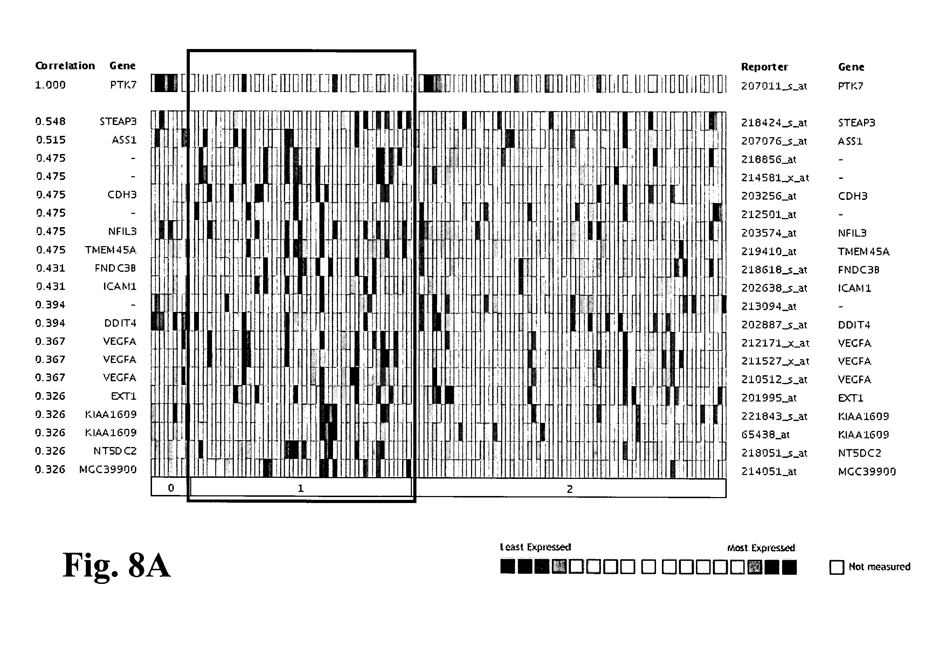

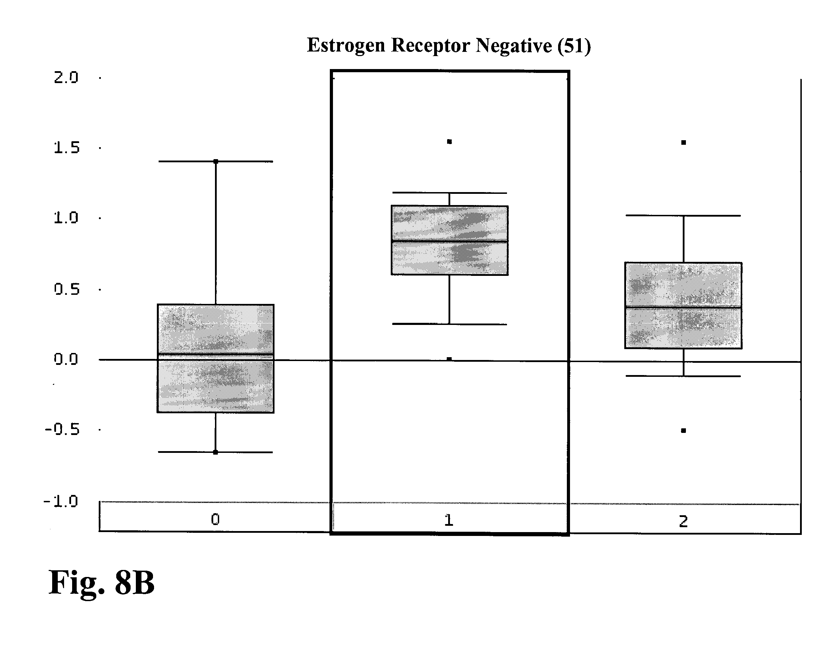

- Figure 8: shows the expression of genes co-expressed with PTK7 in primary breast carcinomas (Hess, Oncomine) which were grouped by ER-status. The colors relate to expression units which are z-normalized to depict relative values within rows. They cannot be used to compare values between rows. A. Hit-map: PTK7 expression in Hess' study of breast cancer gopuped by estrogen receptor status (invasive ductal breast carcinoma), 0: no value (9), 1: estrogen receptor negative (51), 2: estrogen receptor positive (73). B. Statistics: PTK7 expression in Hess' study of breast cancer grouped by estrogen receptor status (invasive ductal breast carcinoma), 0: no value (9), 1: estrogen receptor negative (51), 2: estrogen receptor positive (73).

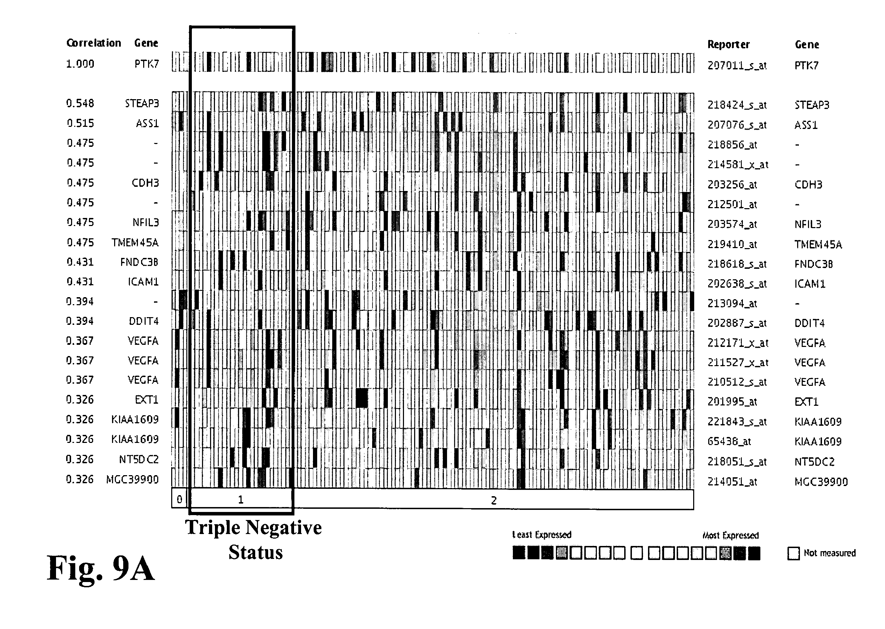

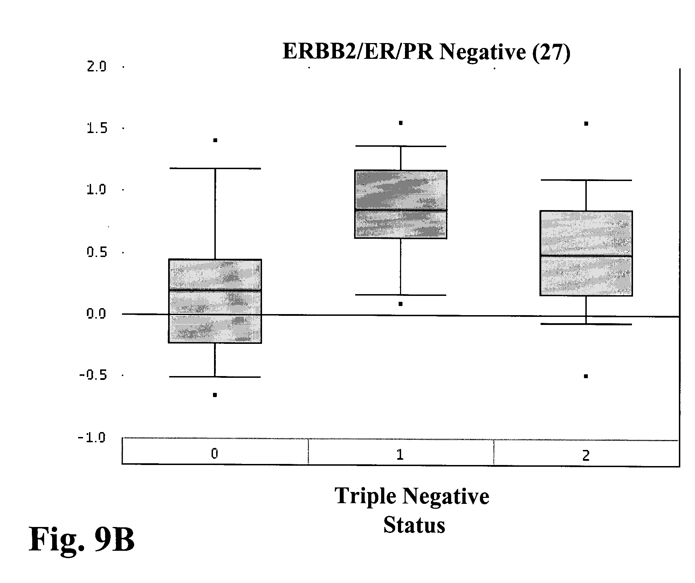

- Figure 9: shows the expression of genes co-expressed with PTK7 in primary breast carcinomas (Hess, Oncomine) which were grouped by triple negative status. The colors relate to expression units which are z-normalized to depict relative values within rows. They cannot be used to compare values between rows. A. Hit-map: PTK7 expression in Hess' study of breast cancer grouped by triple negative status (ErbB2/ER/PR negative), 0: no value (4), 1: triple negative (27), 2: other biomarker status (102). B: Statistics: PTK7 expression in Hess' study of breast cancer grouped by triple negative status (ErbB2/ER/PR negative), 0: no value (4), 1: triple negative (27), 2: other biomarker status (102).

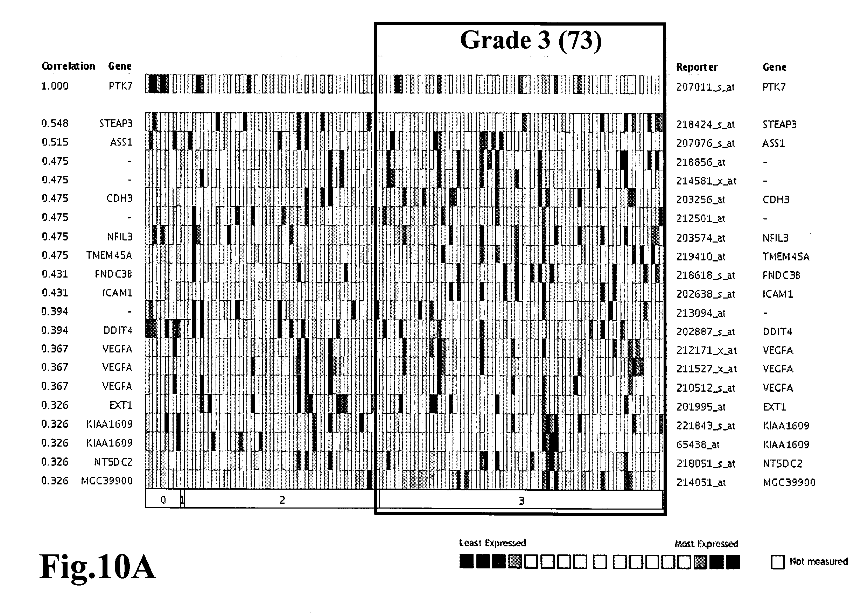

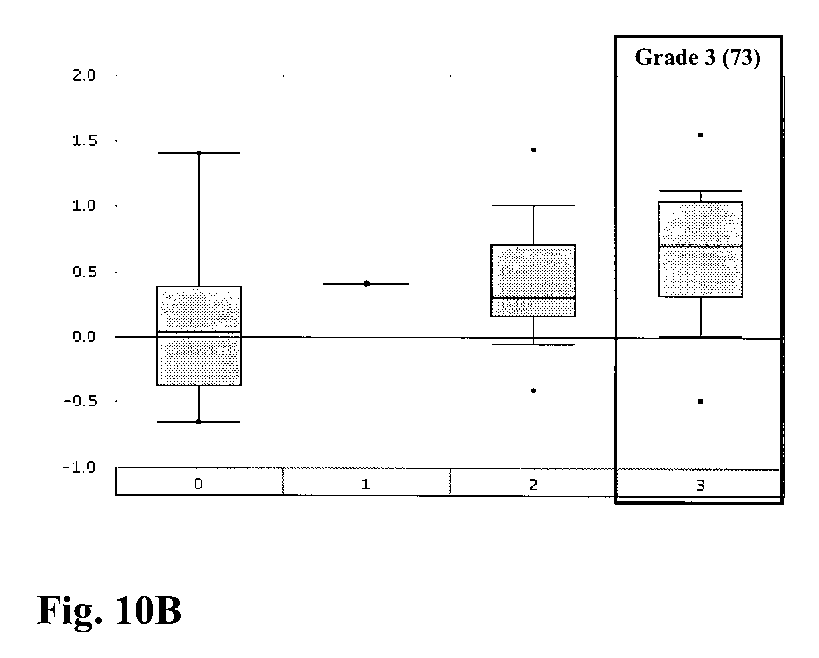

- Figure 10: shows the expression of genes co-expressed with PTK7 in primary breast carcinomas (Hess, Oncomine) which were grouped by grade status. The colors relate to expression units which are z-normalized to depict relative values within rows. They cannot be used to compare values between rows. A: Hit-map: PTK7 expression in Hess' study of breast cancer grouped by grade status (invasive ductal breast carcinoma), 0: no value (9), 1: grade 1 (1), 2: grade 2 (50), 3: grade 3 (73). B: Statistics: PTK7 expression in Hess' study of breast cancer grouped by grade status (invasive ductal breast carcinoma, 0: no value (9), 1: grade 1 (1). 2: grade 2 (50), 3: grade 3 (73).

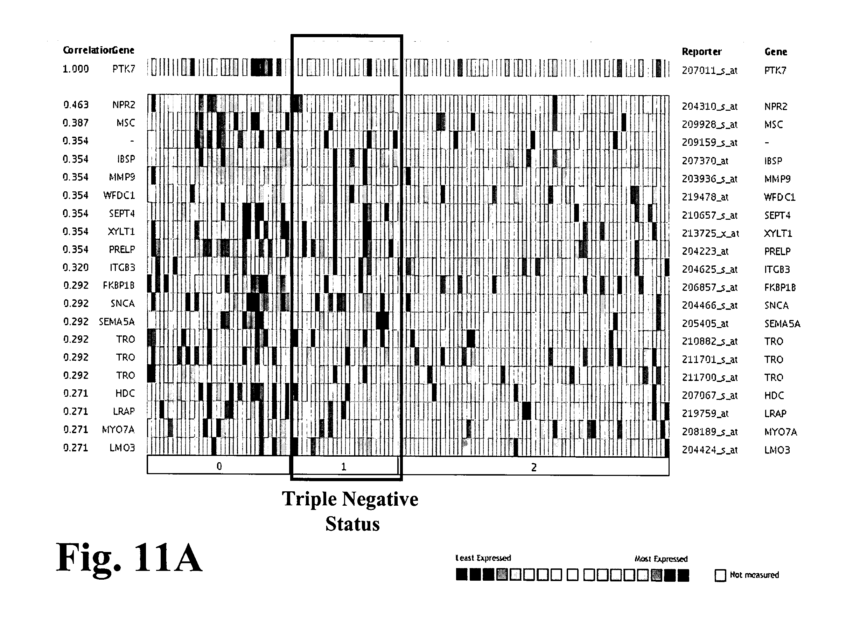

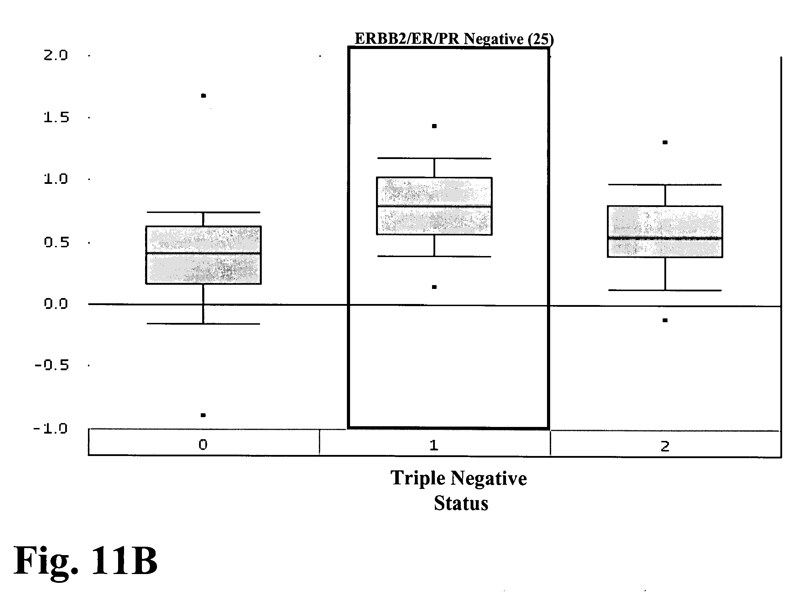

- Figure 11: shows the expression of genes co-expressed with PTK7 in primary breast carcinomas (Minn, Oncomine) which were grouped by triple negative status. The colors relate to expression units which are z-normalized to depict relative values within rows. They cannot be used to compare values between rows. A. Hit-map: PTK7 expression in Minn's study of breast cancer 2 grouped by triple negative status (ErbB2/ER/PR negative), 0 : no value (33), 1: triple negative (25), 2: other biomarker status (63). B: Statistics: PTK7 expression in Hess' study of breast cancer 2 grouped by triple negative status (ErbB2/ER/PR negative), 0: no value (33), 1: triple negative (25), 2: other biomarker status (63).

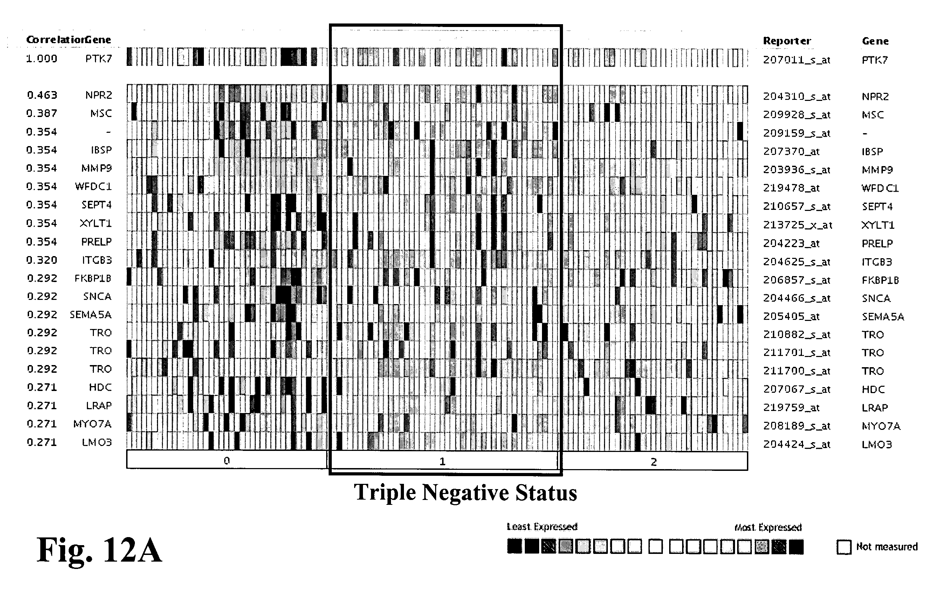

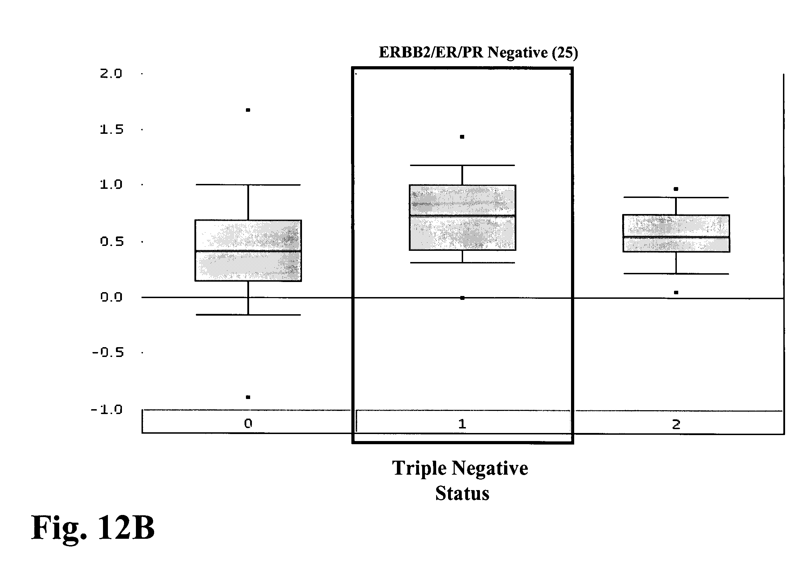

- Figure 12: shows the expression of genes co-expressed with PTK7 in primary breast carcinomas (Minn, Oncomine) which were grouped by bad prognosis status (van't Veer mamma print test or signature). The colors relate to expression units which are z-normalized to depict relative values within rows. They cannot be used to compare values between rows. A. Hit-map: PTK7 expression in Minn's study of breast cancer 2 grouped by bad prognosis status (van't Veer mamma print test or signature), 0: no value (39), 1: bad prognosis (45), 2: good prognosis (37). B. Statistics: PTK7 expression in Minn's study of breast cancer 2 grouped by bad prognosis status (van't Veer mamma print test or signature), 0: no value (39), 1: bad prognosis (45), 2: good prognosis (37).

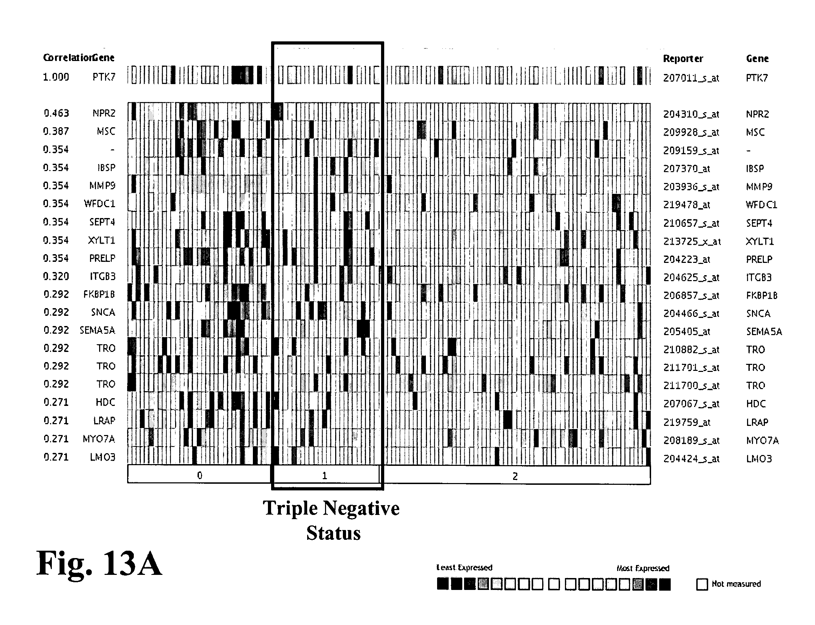

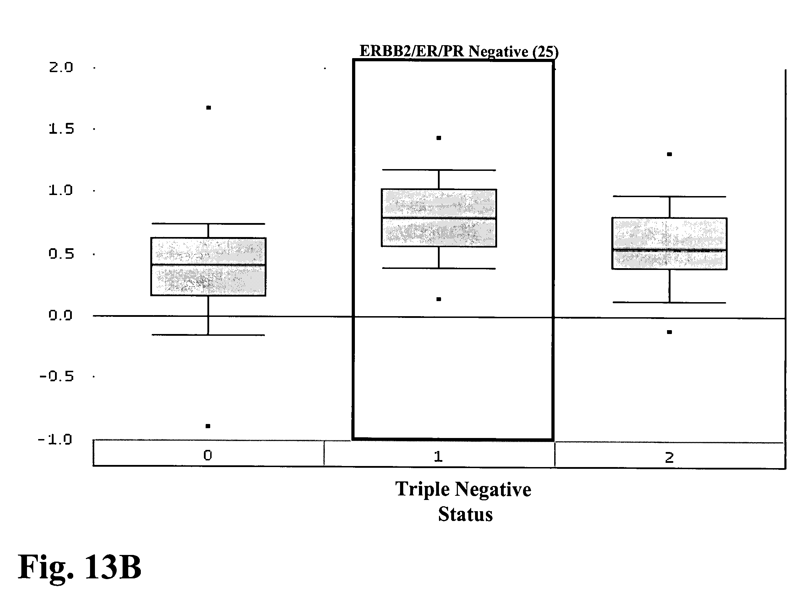

- Figure 13: shows the expression of genes co-expressed with PTK7 in primary breast carcinomas (Wang, Oncomine) which were grouped by estrogen receptor status. The colors relate to expression units which are z-normalized to depict relative values within rows. They cannot be used to compare values between rows. A. Hit-map: ErbB3 (Her3) expression in Wang's study of breast cancer grouped by estrogen receptor status. 1. ER negative (77), 2. ER positive (209). B: Hit-map: PTK7 expression in Wang's study of breast cancer grouped by estrogen receptor status. 1: ER negative (77), 2. ER positive (209).

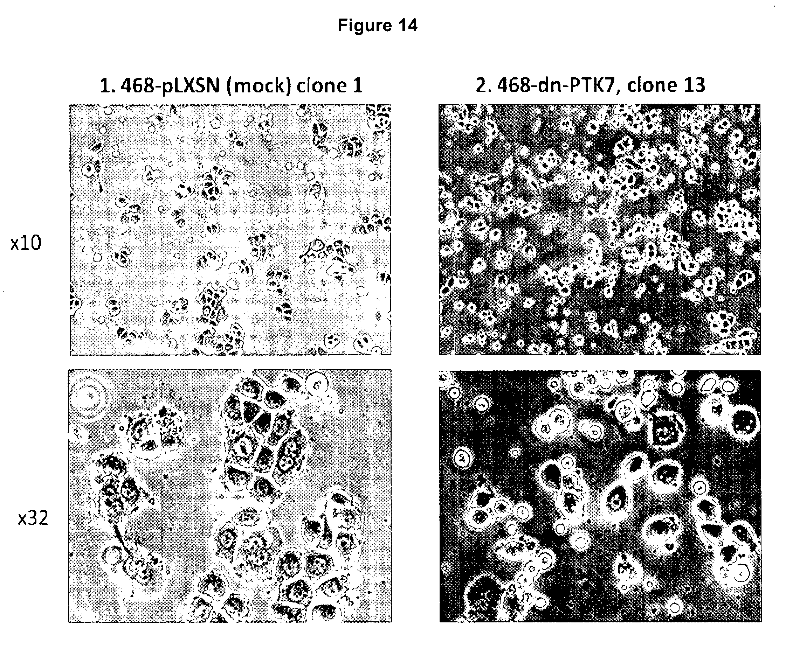

- Figure 14: Morphological characteristics of the triple negative (ER, HER-2, PgR) breast cancer cell line MDA-MB-468 which stably expresses a "dominant-negative" mutant form of the PTK7/CCK4 (dn-CCK4). The names of the cell lines are indicated on the figures. X-10 and X-32 designates the magnification. Cell outgrowth was monitored over the course of the experiment and photographed at 7-14 days using a Zeiss Axiover 35 microscope equipped with OpenLab (UK) digital camera.

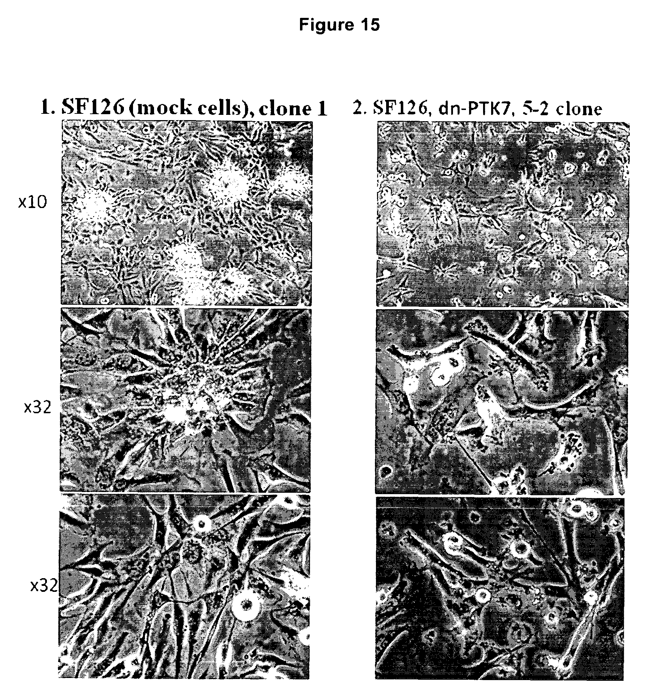

- Figure 15: Morphological characteristics of glioblastoma cell line SF126 which stably expresses a "si-RNA-PTK7". The names of the cell lines are indicated on the figures. X-10 and X-32 designates the magnification. Cell outgrowth was monitored over the course fo the experiment and photographed at 7-14 days using a Zeiss Axiover 35 microscope equipped with OpenLab (UK) digital camera.

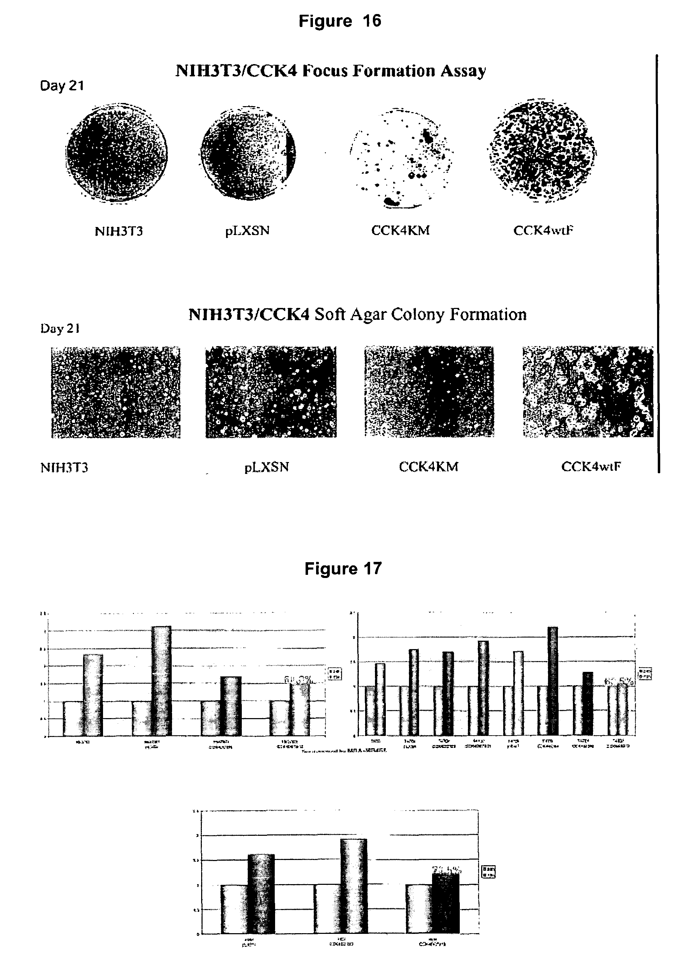

- Figure 16: illustrates how NIHT3 cells gain the ability to form foci as well as colonies in soft agar after transfection with PTK7.

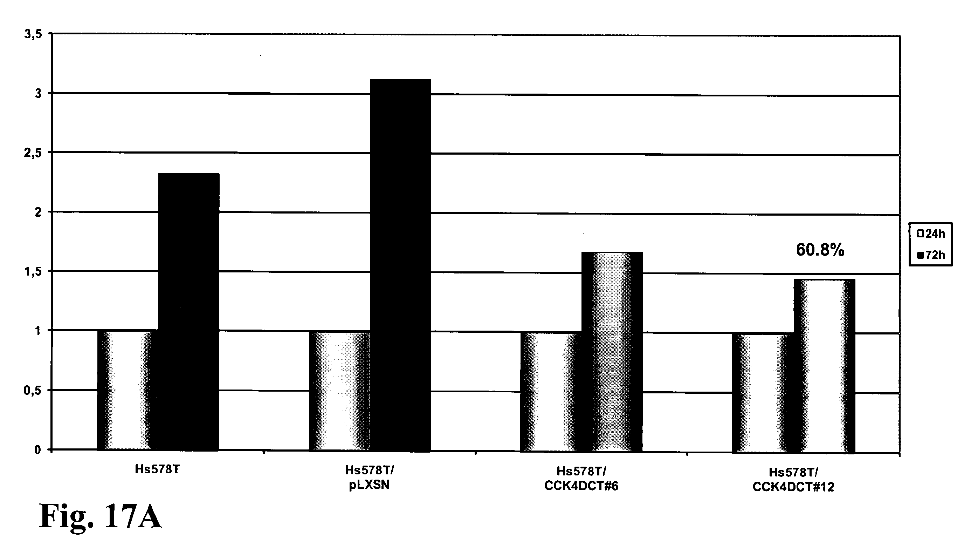

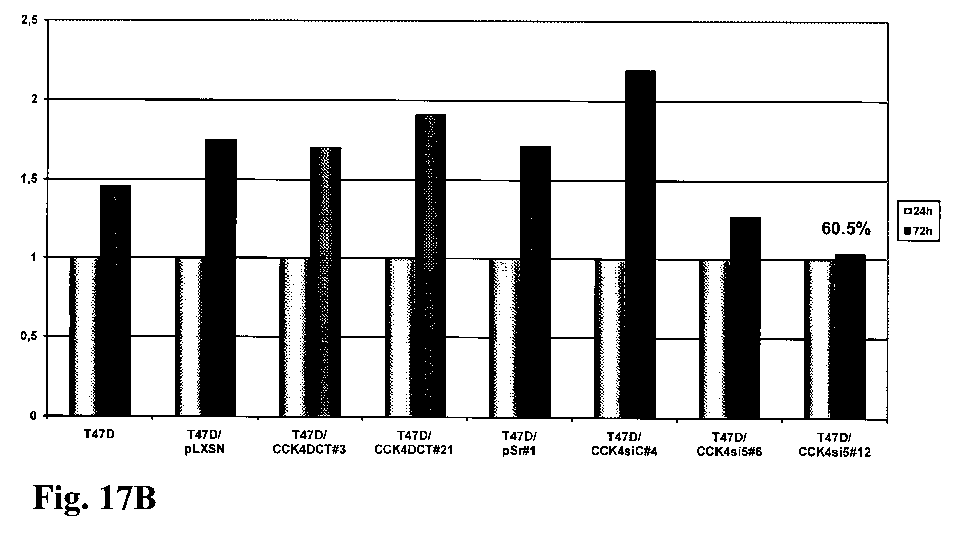

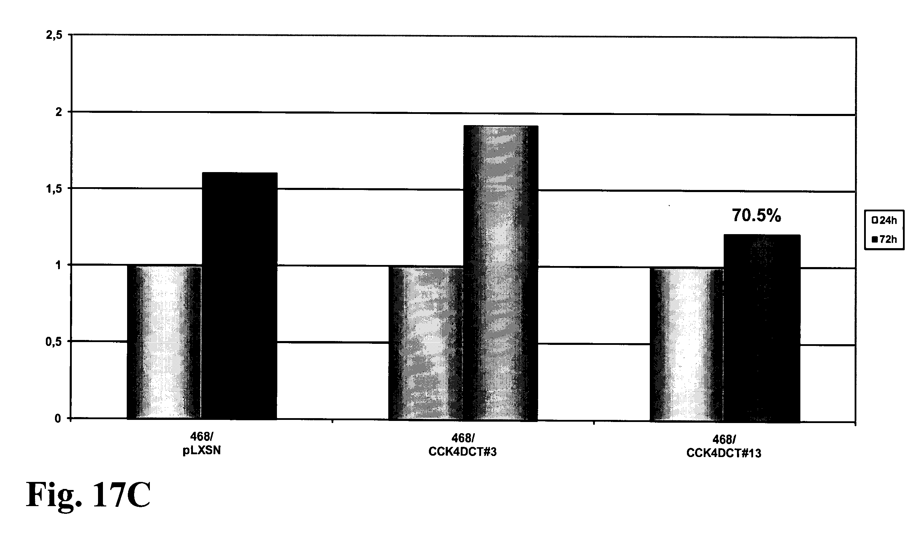

- Figure 17: illustrates the cell proliferation rate of cell lines which were stably transfected with either PTK7 wild type, dominant negative PTK7 or a PTK7 siRNA.

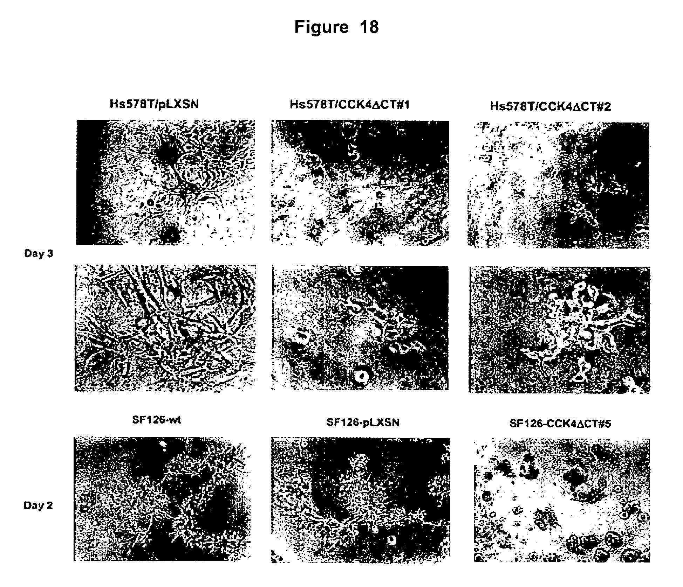

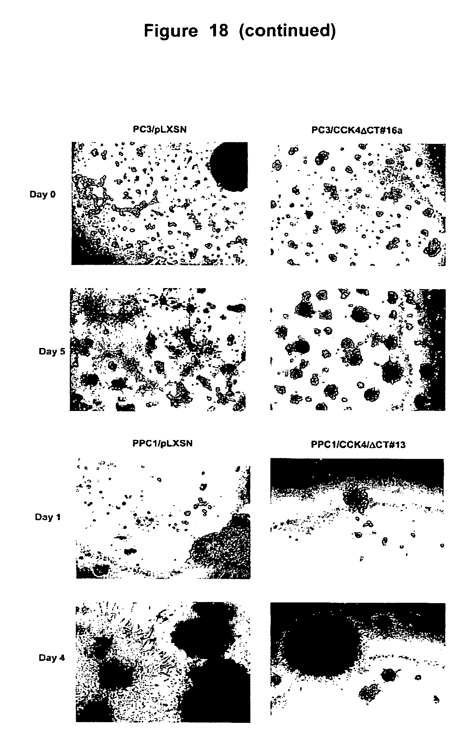

- Figure 18: illustrates that the ability to form outgrowth in matrigel is impaired in Hs578T, SF126, PC3 and PPC1 cells stably expressing dominant negative PTK7 (PTK7ΔCT)

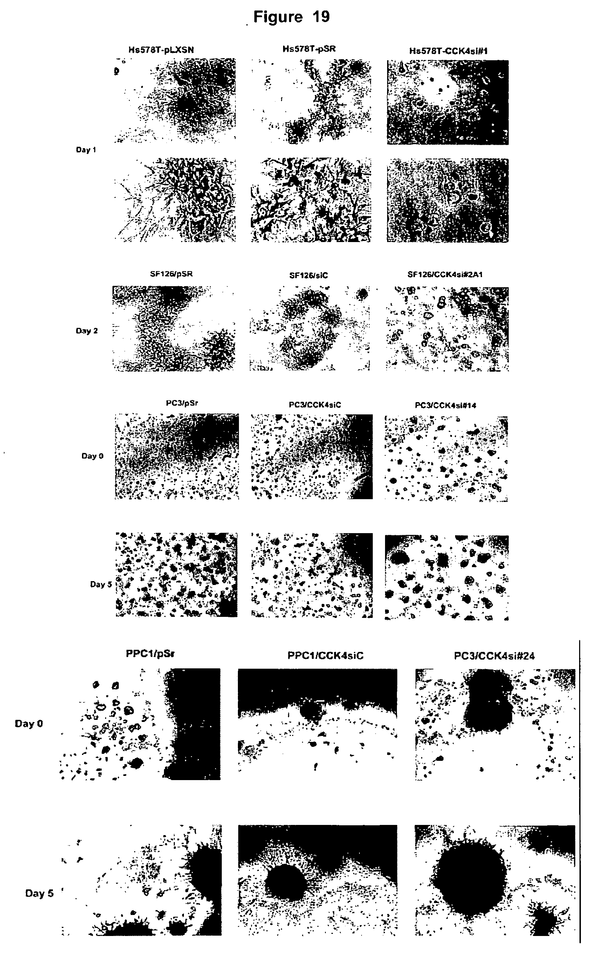

- Figure 19: illustrates that the ability to form outgrowth in matrigel is impaired in Hs578T, SF126, PC3 and PPC1 cells stably expressing PTK7 siRNA constructs.

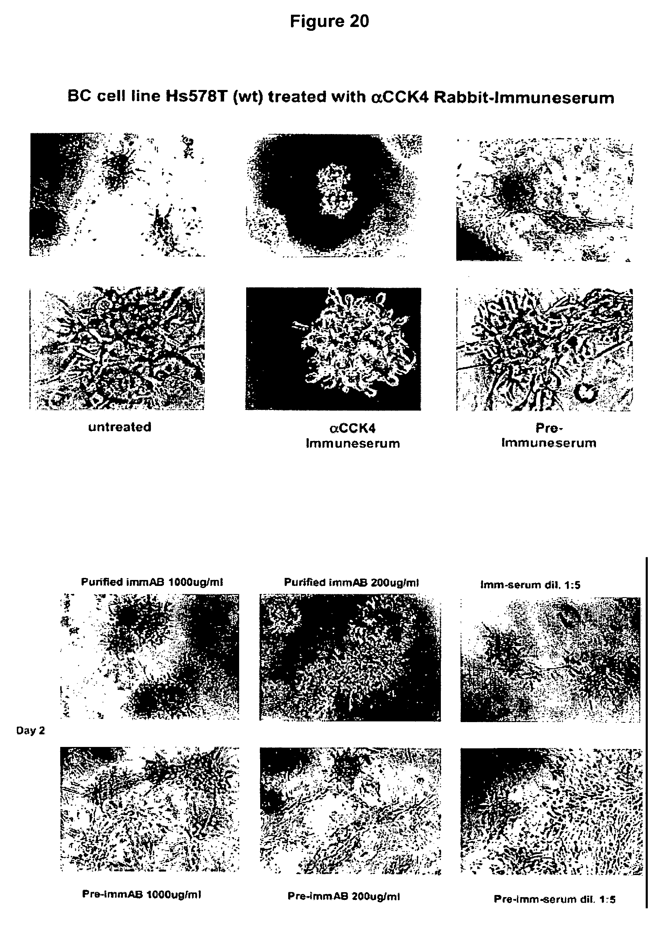

- Figure 20: illustrates that the ability to form outgrowth in matrigel is impaired in Hs578T which are treated with polyclonal immune serum raised against the extracellular domain of PTK7.

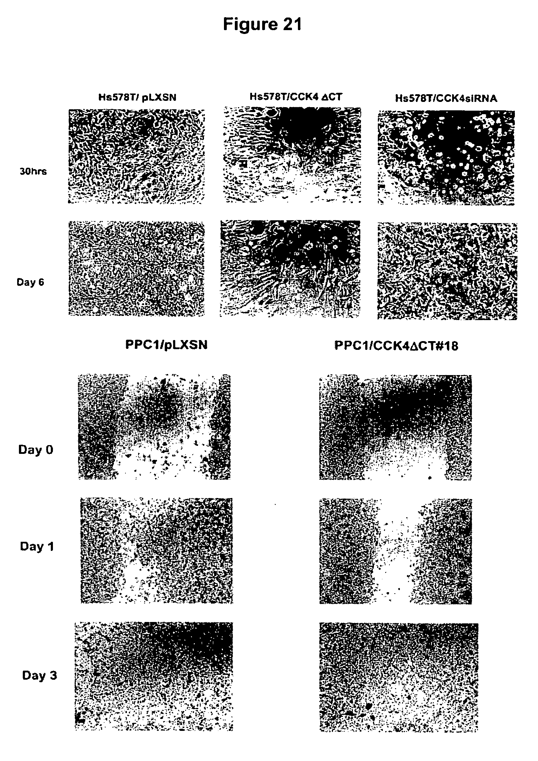

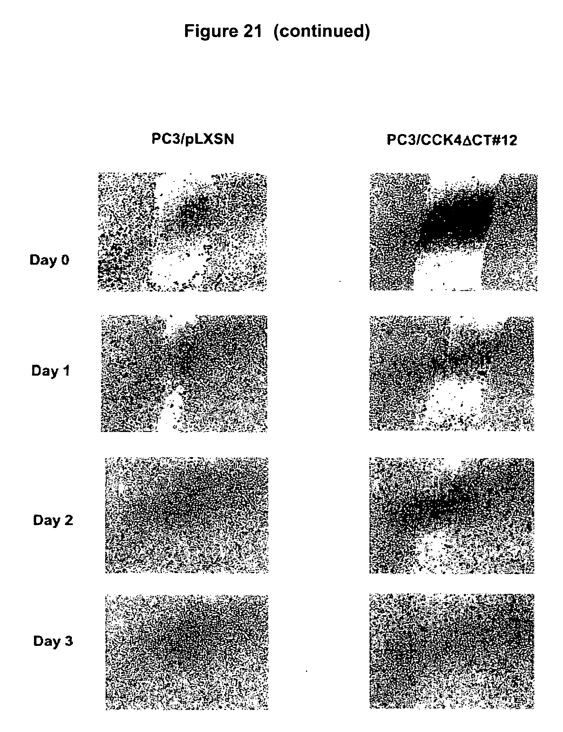

- Figure 21: illustrates that Hs578T, PC3 and PPC1 cells which are stably expressing dominant negative PTK7 (PTK7deltaCT) wound closure is delayed.

### Examples

### Example 1: Patient population, tumor samples and cell lines

This study was approved by the Red Cross Clinic Review Boards of the Munich University. Tissues from 35 breast carcinomas (BC) patients, normal tissues and blood pellets were collected at the time of surgery from January 2006 to January 2009 at the Red Cross Clinic. All the primary tumor sections were evaluated by a study pathologist and clinical information was collected (not shown). All patient identifiers were coded to protect confidentiality. All tissues material and blood cells were stored in boxes and tubes at -80°C until use.

The median age of patients at the time diagnosis was 55 years (range 29-81) and most of them were postmenopausal. Tumours were classified according to the WHO histological typing of breast tumours: ductal carcinomas, lobular carcinomas, mixed ductal-lobular carcinomas and modularly carcinomas. Pooled "normal" cDNA derived from normal breast mRNAs was used as control and for normalisation.
Expression profiles of protein kinases (PK) and others cancer related genes in "normal" cDNAs mentioned above were evaluated separately. In this study we also included 21 BC and 3 normal breast epithelial cell lines. The sources of the breast cancer: cell lines were as follows: BT-20, BT-474, BT-483, BT-459, Du-4475, MDA-MB-134, -157, -175, -361, -436, -453, -468, SK-BR-3, and ZR-75-1, T-47D, MDA-MB-231, ZR-75-30 were obtained from the American Type Culture Collection (ATCC, Rockville, Md.). MCF-7, clone and BC cell line DAL were supplied by SUGEN (Redwood City, Calif.). The HBL-100 cell line was from ATCC. This cell line was derived from normal tissue but contains tandemly integrated SV-40 sequences (9). Cultures were maintained in exponential growth in RPMI 1640 medium, supplemented with 6 mM glutamine, 10 .mu.g/ml human insulin and 10% Foetal calf serum (FCS) (CSL, Parkville, Australia). Normal breast epithelial cell strains MCF10A, MCF10 T-24 and MCF10 neo were provided by Dr. B. Gilles (Arizona Cancer centre). Ac745 was provided by Dr. M. Stampfer and grown in the DMEM F12 medium supplemented by the condition medium of Hs578Bst, Insulin, Hydrocortisone, EGF, Cholera toxin, vitamins and antibiotics. Cells were free from Mycoplasma contamination.

### Example 2: Isolation and Fractionation of RNA and DNA

Total RNA and genomic DNA was isolated from the same cell pellet by lysis in guanidinium isothiocyanate solution (GTS buffer: 4 M guanidinium isothiocyanate, 25 mM sodium citrate pH 7.0, 0.5% Sarkosyl, and 0.1 M: .beta.-mercaptoethanol) followed by phenol-chloroform extractions. Total RNA was isolated using standard methods [Sambrook et al. 1989] with modifications. DNA was collected and extracted twice with an equal volume of phenol: chloroform: isoamylalcohol (25:24:1). RNA and DNA were isolated from each cell line on a minimum of 3 independent occasions. Total and mRNA integrity and cDNA complexity was controlled by agarose gel electrophoresis and Northern blots using specific probes. Some mRNA extraction was performed using the OligoTex mRNA isolation Kit (Quiagen Biotech, Germany). Cell pellets were resuspended in lysis/binding buffer, vortex-mixed briefly, passed three times through a 21 G needle and applied to a spin lysate column and centrifuged at 13,000 g for 3 min. The lysate was then mixed gently with Oligo-dT cellulose (Stratagene Inc.) and applied to a pre-wetted Oligotex molecular biology column (Quagen Biotech). The column was washed three times with lysis/binding buffer and four times with wash buffer before eluting the mRNA with pre-warmed (65 C) elution buffer. The quantity of mRNA was measured using the OD260. For cDNA synthesis were applied 5ug of mRNA using Smart-cDNA synthesis protocol (Clontech Inc, USA) with our modification.

### Example 3: Cluster analyzes

### cDNA array preparations

PK and PP gene expression was analyzed by hybridization on nylon filters arrays with radioactive targets (cDNA). The arrays contained 1645 genes encoding kinases, phosphatases and, others signalling proteins: ligands, adaptors, transcription factors, metalloproteinases/ADAMs, apoptosis related genes and 11 housekeeping genes (the list is available at http://www.biochem.mpg.de or ullrich@biochem.npg.de). Their identity was verified by sequencing of plasmid DNA and compared with GenBank sequence information. Identity of PK and PP was conformed for all clones spotted on nylon filters ones, or in duplicate. For normalisation purpose, the GFP gene was spotted two times as well as genomic and vector DNA. Purification of plasmids was done using a plasmid purification kit (Qiagen, Germany).

### cDNA array hybridization

Filters were initially pre-washed in 0.5% SDS for 5 min, with agitation. In 10 ml of the pre-hybridisation solution was included Yeast tRNA. Human Cot-1 DNA (BRL/Life technologies) was used in the hybridization step which was performed in a Roller bottle (Hybaid Inc.) for 16 h in a roller oven at 65°C Labelled probe was denatured for 10 min at 100°C and then placed immediately into the hybridisation mixture which was incubated for a further 18 h at 65 degree of centigrade after 18 h, the hybridisation mixture was discarded and the array was washed twice in 2 sodium chloride: sodium citrate (SSC) buffer, 0.2% SDS for 20 min at 42°C. with continued rotation in the incubator. A third wash was performed in 0.2.times.SSC, 0.1 % SDS for 15-60 min at 65°C in a plastic box with horizontal shaking. After the third wash, the filter was placed on a piece of moistened Whatman paper and covered with Saran wrap. The array was then placed into an image cassette with a Phosphorimager storage screen (Fuji, Japan) and exposed for 2 days.

### Image acquisition and analysis

Exposed phospho-imager storage screens were scanned once on a Phosphoimager Scanner (Fuji) at a resolution of 50 microns: and were visualised using MacBAS 2000 (Fuji). Images were imported into ArrayVision V (Canada) for analysis by a software protocol. Mapping of individual elements to an internal reference database was achieved by aligning the images onto a software-based matrix using a total-of 4 control elements representing total genomic control DNA, GFP, and vector. Normalisation was performed by multiplying the raw intensity for each data element by a normalisation factor equal to the average raw intensity for all the vector elements divided by 100 (this value is the average raw intensity for all elements, derived from a large number of different hybridizations performed by us the development of the arrays). Software-based pair-wise comparisons of the normalised images were made against the image obtained from hybridisation of labelled cDNA taken from pooled "normal" cDNA derived, from normal breast RNAs, immortal (preneoplastic) breast epithelial cell lines, as indicated above. Changes in expression levels were calculated using normalised intensities and given as ratios (positive ratios indicated an increase in transcript levels, negative ratios indicated a decrease in transcript levels) and were visualised by Scatter-blot graphics and TreeView program (M. Eisen).

### Array data analysis

Before analysis of the results, the reproducibility of the experiments was verified by comparing duplicate spots or hybridizations with the same cDNA on independent arrays, or two independent hybridizations with cDNA prepared from the same RNA. In each case, the results showed good reproducibility with respective correlation coefficients 0.96, 0.98 and 0.98 (data not shown). The reproducibility was sufficient enough to consider a 2-fold expression difference as significantly differential. Subsequent analysis was done using Excel and statistical software. The search for genes with expression levels correlated with tumour parameters was done in several successive steps. First, genes were detected by comparing their median expression level in the two subgroups of tumours differing according to parameters of interest. We used the median values rather than the mean values because of the high variability of the expression levels for many genes, resulting in a standard deviation expression level similar: or superior to the mean value and making comparisons with means impossible. Second, these detected genes were inspected visually on graphics and, finally, an appropriate statistical analysis was applied to those that were convincing to validate the correlation. Comparison of PTK7-CCK4 expression between ER-positive tumours and ER-negative tumours was validated using a Mann-Witney test. Correlation coefficients were used to compare the gene expression levels with the number of axillary nodes involved.

### Cluster analysis

The data from this study were analyzed and displayed as described (Ref. Eisen et al and TIGR). Briefly, a hierarchical clustering algorithm produces a table of results wherein the elements/cDNAs of the array (representing specific genes) are grouped together based a similarities in their patterns of gene expression.
The same algorithm is applied to cluster the experimental samples (i.e., cell lines and tumours) according to the similarities in their overall patterns of gene expression. The data tables, thus ordered, are presented graphically as colours images. Along the vertical axis, the genes analyzed are arranged as ordered by the clustering algorithm, so that the genes with the most similar patterns of expression are placed adjacent to each other. Along the horizontal axis, experimental samples are similarly arranged such that those with the most similar patterns of expression across all genes are placed adjacent to each other. The colour of each cell/square in this tabular image represents the measured expression ratio of each gene in question. The colour saturation is also directly proportional to the magnitude of the measured gene expression ratio with the brightest red squares having the highest T/N ratio (i.e., >8-fold difference), the brightest- green squares having the lowest T/N ratio, black squares indicating a ratio of approximately 1, and grey squares indicating insufficient data quality.

### Results

PTK7 is expressed in a variety of primary breast tumour tissues as well as in breast cancer cell lines.
The expression of PTK7 in primary breast cancer cells was significantly higher than in normal breast tissues and lymph nodes (LN) from the same patients (red high and green low, subsequently), shown in (Fig.1). However, in some patients using gene expression profiling (array hybridization) of primary breast cancer tumours, normal tissues and lymph nodes (LN) shows an increasing PTK7 in LN, indicating about possible dissemination of a cancer cell in the lymph system (see Patient 20). The verification of expression PTK7 in selected breast cancer patient by PCR shows elevation of the PTK7 transcripts in LN which were with epithelial cancer cells (Fig.2). Highest expression of the PTK7 in LN (lympf nodes) with dissemination cancer cells in comparison with primary tumours indicates about significance this receptor in metastasis development. The expression of PTK7 in breast cancer and normal breast cell lines revealed highest expression of the PTK7 in a basal like cell lines, which is strongly expressed EGFR and don't have ER and HER2 (shown below in Fig.3). 3 normal breast cell lines used in our studies belong to basal phenotype and don't expressed ER and HER2 and expressed relatively high level of PTK7 (Ac745, MCF10a and Hs578Bst).

PTK7 is expressed in a variety of primary breast tumour tissues as well as in breast cancer cell lines. The expression in breast cancer cell lines and primary cancer cells was significantly higher than in normal cells, which have a low expression level of PTK7. Expression in lymph node tissue and cell lines had similar levels as in the normal cells (Fig.1), The results indicate that the expression of PTK7 is elevated in primary breast cancer as well as in breast cancer cell lines. On this Fig. 1 there is no cancer or normal cell lines. This is a primary tissues material. The ID and origin of the primary tumours were as follows: PrimaryBC (PrBC), 33TK (our patients No and abbreviation of names and family names), pT3N2aM0 (WHO tumour classification), G3 (grade of tumour), ER12 (estrogens receptor value), most of this information has submitted by pathologist.

### Example 4: PCR evaluation of PCK7 expression in primary breast cancer

A PCR mixture was prepared containing 2.0 µl of 10xPCR buffer, 2 µl of 20 mM primer solutions of CCK4 forward primer Ex7 (5'-GGA AGC CAC ACT TCA CCT AGC AG-3') and reverse primer Ex11 (5-CTG CCA CAG TGA GCT GGA CAT GG-3'), and 2.5 U RedTaq DNA polymerase (Sigma Inc., USA), 2.0 µl of dNTPs and H₂O up to 20 µl. For the control PCR of alpha-tubulin the forward primer (5'-AAG TGA CAA GAC CAT TGG GGG AGG-3') and the reverse primer (5'-GGG CAT AGT TAT TGG CAG CAT C-3') were used.
The PCR-program started with a denaturation and enzyme activation step at 94°C for 1 min, followed by 32 cycles of denaturation at 94°C for 30 s, primer annealing at 57°C (for primer CCK4) or 62°C (for primers Tubulin) for 30 s, and extension at 72°C (for primers of both genes) for 1 min, followed by a final extension step at 72°C for 5 min.

The PCR products were analyzed by separation on a 2% agarose gel which was stained with ethidium bromide.

### Results

The PCR products had a size of approximately 800bp. The results show that a baseline expression at mRNA level is present in all cells (Fig. 2).

The lymph nodes of the indicated patients have been fully infiltrated with disseminated cancer cells where expression of PTK7 was higher as in a primary breast cancer cell. This means that PTK7 is significant for the metastasizing cancer cells and thus is a marker and a target for the treatment of triple negative (ER-, HER2-, PgR-) breast cancer cells and basal-like breast cancer cells.

### Example 5: Analyzes of Oncomine data, data sorting

The Oncomine (www.oncomine.org) database tool was used to analyze mRNA expression microarray data from several breast cancer studies (Meta-analysis of gene expression of the PTK7 and HER3 as cancer targets). The gene expression level of PTK7 and HER3 was studied using our microarray platform and Oncomine. Briefly, PTK7 gene was queried in the database, and the results were filtered by selecting breast carcinomas. The data from study classes of benign vs. cancer were used for hit-maps and box plots. P values for each group were calculated using student t-test. Standardized normalization techniques and statistical calculations are provided on the Oncomine website. Standard analyses were applied to raw microarray data using Robust Multichip Average for Affymetrix data and Loess for cDNA arrays. If not already performed by the author of the original study, reference data was incorporated at this step. Oncomine then applies a z-score normalization to scale data and allows comparison of multiple independent studies. Normalization includes log₂ transformation and setting the array median to 0 and standard deviation to 1. Fold change was calculated by dividing the average of the normalized tumor values by the average of the normalized normal tissue values (Rhodes et al., Proc Natl Acad Sci USA 2004; 101 (25):9309-14; Rhodes et al., Neoplasia, 2004; 6(1):1-6).

### Results

PTK7 has a higher expression in breast cancer cells which lack the expression of the estrogen receptor compared to breast cancer cells which do express it (Fig. 3). The expression of PTK7 and EGFR is higher in BC cells which have a low Her2/Her3-expression and lack ER, while the expression is low in ER-positive BC cells with a high Her2/Her3 expression (Fig.4). The expression of genes which are co-expressed with PTK7 is elevated in TNBC cells when compared to other biomarkers (Figs. 5 and 6). In primary BC cells, the PTK7 expression was higher in ER-negative cells than in ER-positive (Fig. 7A), and the survival rate after five years was lower in patients which had a high PTK7 expression (Fig. 7B). The expression of genes which are co-expressed with PTK7 is elevated in ER-negative BC cells (Fig. 8A and B) and TNBC cells (Fig. 9A and B) when compared to other biomarkers. The expression of PTK7 was higher in primary BC cells which had a higher grade (Fig. 10A and B). PTK7 expression was higher in primary TNBC cells (Fig. 11A and B) and was also correlated with a poor prognosis (Fig. 12A and B). The expression of ErbB3 was low in cells which were ER-negative and high in cells which were ER-positive, and thet PTK7 expression was high in ER-negative cells while it was low in ER-positive cells (Fig. 13).
Summing it up, the expression of PTK7 is predominantly over-expressed in TNBC and is also correlated with a poor prognosis.

### Example 6: PTK/NIH3T3 focus formation assay.

NIH3T3 cells (mouse) were stably infected with different pLXSN/PTK7 constructs using supernatants of the ecotropic retrovirus producing cell line PhoenixE and selected with G418. After verification of PTK7 expression in Western Blots cells were propagated and used for several different cellular assays.
For Focus Formation Assays 2x10⁵ cells were seeded into 6cm-dishes and left to grow for 21 days with changing of media every two to three days. Cells were then stained and fixed with 0.5% Cristal Violet/20% Methanol.

### Results

PTK7 over-expressing NIH3T3 cells gain the ability to form foci as well as colonies in soft agar. In CCK4wt cells a large amount of foci could be detected, whereas untransfected and mock-transfected cells showed no focus formation. The fact that NIH3T3 cells gain the ability to grow independently of their attachment to a tissue culture surface strongly indicates the oncogenic potential of PTK7/CCK4 (Fig. 15).

### Example 7: Proliferation rate of cell lines, stably transfected with dominant negative PTK7 and PTK7 siRNA

Hs578T, T47D and MDA-MB468 cells which show a high endogenous expression level of PTK7 were stably infected with CCK4ΔCT and CCK4si constructs using supernatants of the amphotropic retrovirus producing cell line PhoenixA which had been transiently transfected with pLXSN or pSUPERretro constructs. After selection with G418 for 14 days, clones were picked, propagated and checked for PTK7 expression in Western Blots. Cells were then used for several different cellular assays.
For proliferation assays cells were seeded at 1x10⁴ cells per well into 96well dishes and left to proliferate for 24 and 72 hours. After 24 and 72 hours viable cells were detected using the CellTiter-Glo Luminescent Cell Viability Assay (Promega), which is based on the quantitation of ATP, signaling the presence of metabolically active cells.

### Results

Cell lines stably over-expressing dominant negative PTK7 or PTK7 siRNA tend to proliferate more slowly than control cells. This is another indication for the oncogenic potential of PTK7 in triple negative (ER-, HER2-, HER3-) breast cancer cells (Fig. 17).

### Example 8: Outgrowth in matrigel of cell lines, stably transfected with dominant negative PTK7 and PTK7 siRNA

Hs578T, SF126, PC3 and PPC1 cells were stably infected with CCK4ΔCT and CCK4si constructs using supernatants of the amphotropic retrovirus producing cell line PhoenixA which had been transiently transfected with pLXSN or pSUPERretro constructs. After selection with G418 for 14 days clones were picked, propagated and checked for PTK7 expression in Western Blots. Cells were then used for several different cellular assays.
For matrigel assays cells were seeded at 2.5x10⁴ cells per well into 96well dishes, which had previously been coated with 3% Matrigel. Colony outgrowth was visualized after two or three days with a Zeiss Axiovert S100 microscope.

### Results

The ability form outgrowth in matrigel is impaired in cell lines stably over-expressing dominant negative PTK7 or PTK7 siRNA (Figs. 18, 19). This is another indication for the oncogenic potential of PTK7 in triple negative (ER-, HER2-, HER3-) breast cancer cells.

### Example 9: Outgrowth in matrigel of Hs578T cells, treated with a polyclonal antibody raised against the extracellular domain of PTK7

Hs578T, SF126, PC3 and PPC1 cells were stably infected with CCK4ΔCT and CCK4si constructs using supernatants of the amphotropic retrovirus producing cell line PhoenixA which had been transiently transfected with pLXSN or pSUPERretro constructs. After selection with G418 for 14 days clones were picked, propagated and checked for PTK7 expression in Western Blots. Cells were then used for several different cellular assays.
For matrigel assays cells were seeded at 2.5x10⁴ cells per well into 96well dishes, which had previously been coated with 3% Matrigel. Colony outgrowth was visualized after two or three days with a Zeiss Axiovert S100 microscope.

### Results

The ability form outgrowth in matrigel is impaired in Hs578T cells, which were treated with a polyclonal antibody against the extracellular domain of PTK7 (Fig. 20). This indicates that the antibody effectively inhibits the activity of PTK7 and thereby may be an effective tool especially for the inhibition of metastasis of triple negative (ER-, HER2-, HER3-) breast carcinomas.

### Example 10: Wound healing assay

Hs578T, PC3 and PPC1 cells were stably infected with CCK4ΔCT constructs using supernatants of the amphotropic retrovirus producing cell line PhoenixA which had been transiently transfected with pLXSN constructs. After selection with G418 for 14 days clones were picked, propagated and checked for PTK7 expression in Western Blots. Cells were then used for several different cellular assays.
For Wound Healing Assays cells were seeded at 2x10⁵ cells per well into 24well dishes. After the monolayers had reached near confluency, they were wounded with a uniform scratch. Cells were washed twice, and were permitted to migrate into the wound area for several days. Wound closure was visualized with a Zeiss Axiovert S100 microscope.

### Results

In Hs578T, PPC1 cells stably expressing dominant negative PTK7 wound closure is delayed (Fig. 21). These results further indicate that PTK7 is involved in the invasion and proliferation of triple negative (ER-, HER2-, HER3-) breast cancer cells.

In the present examples 1 - 10 all tested cell lines were triple negative (ER, HER-2, PgR) except the T47D breast cancer cell line which was triple positive (ER+, HER2+, PrR+).

## Claims

**1.** Pharmaceutical composition for the treatment and prophylaxis of angiogenesis, invasion and metastatic outgrowth of the breast cancer subtypes triple negative (ER, HER-2, PgR) breast cancer and basal-like breast cancer comprising an agent which interacts with or modulates the expression or activity of a PTK7 polypeptide.

**2.** Pharmaceutical composition according to claim 1, wherein the triple negative breast cancer is **characterized by** the three negative receptors ER, PgR and HER2 and wherein the basal-like breast cancer is **characterized by** the two negative receptors ER and HER2 and at least one positive receptor of the group consisting of CK5/6, CK14, CK17 and EGFR.

**3.** Pharmaceutical composition according to claim 1, wherein the agent is able to downregulate the expression of the PTK7 polypeptide and / or wherein the agent is able to decrease the activity of the PTK7 polypeptide.

**4.** Pharmaceutical composition according to claim 1, wherein the agent is an antibody against PTK7 or a functionally active fragment, derivative or analogue of that antibody.

**5.** Pharmaceutical composition according to claim 1, 2, 3 or 4, wherein the agent is selected from or wherein the antibody is conjugated with a taxane, abrin, ricin A, pseudomonas exotoxins, diphtheria toxins, tumour necrosis factor, alpha-interferon, β-interferon, nerve growth factor, platelet derived growth factor, tissue plasminogen activator, thrombotic agents, anti-angiogenic agents, angiostatins, endostatins, lymphokines, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF), mechlorethamine, thioepa chlorambucil, melphalan, carmustine, lomustine, cyclothosphamide, busulfan, dibromomannitol, streptozotocin, mitomycin C, cis-dichlorodiamine platinum (II), cytoskeletal disruptors, inhibitors of topoisomerase II, anthracyclines, daunorubicin, platinum-based agents, retinoids, vinca alkaloids and derivatives, antimetabolites, methotrexate, 6-mercaptopurine, 6-thioguanine, cytarabine, 5-fluorouracil decarbazine, paclitaxel, trastuzumab, capecitabine, letrozole, exemestane, anastrozole, lapatinib, doxorubicin, cyclophosphamide, fluorouracil, docetaxel, methotrexate, gemcitabine, epirubicin, vinorelbine, fulvestrant, toremifene, protein-bound paclitaxel, testolactone, bevacizumab, cytochalasin B, gramicidin D, ethidium bromide, emetine, mitomycin, etoposide, tenoposide, vincristine, vinblastine, colchicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, dactinomycin, bleomycin, mithramycin, anthramycin, calicheamicins, duocarmycins, alkylphosphocholines, topoisomerase I inhibitors, taxoids or suramins.

**6.** Pharmaceutical composition according to claim 1, 2, 3, 4 or 5, wherein the agent is a combination of a DNA damaging agent and a PARP inhibitor.

**7.** Use of an agent which interacts with or modulates the expression or activity of a PTK7 polypeptide for the manufacture of a pharmaceutical composition for the treatment and prophylaxis of angiogenesis, invasion and metastatic outgrowth of triple negative breast cancer or of basal-like breast cancer.

**8.** Use according to claim 7, wherein the agent is an antibody against PTK7 or a functionally active fragment, derivative or analogue of that antibody or wherein the agent is a combination of a DNA damaging agent and a PARP inhibitor.

**9.** Use according to claim 8, wherein the antibody is monoclonal, polyclonal, chimeric, humanized or bispecific, or is conjugated to a therapeutic moiety, to a detectable label, to a second antibody or to a fragment of that second antibody, to a cytotoxic agent or to a cytokine.

**10.** Use according to claim 7, 8 or 9, wherein the agent is selected from or wherein the antibody is conjugated with a taxan, abrin, ricin A, pseudomonas exotoxins, diphtheria toxins, tumour necrosis factor, alpha-interferon, β-interferon, nerve growth factor, platelet derived growth factor, tissue plasminogen activator, thrombotic agents, anti-angiogenic agents, angiostatins, endostatins, lymphokines, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF), mechlorethamine, thioepa chlorambucil, melphalan, carmustine, lomustine, cyclothosphamide, busulfan, dibromomannitol, streptozotocin, mitomycin C, cis-dichlorodiamine platinum (II), cytoskeletal disruptors, inhibitors of topoisomerase II, anthracyclines, daunorubicin, platinum-based agents, retinoids, vinca alkaloids and derivatives, antimetabolites, methotrexate, 6-mercaptopurine, 6-thioguanine, cytarabine, 5-fluorouracil decarbazine, paclitaxel, trastuzumab, capecitabine, letrozole, exemestane, anastrozole, lapatinib, doxorubicin, cyclophosphamide, fluorouracil, docetaxel, methotrexate, gemcitabine, epirubicin, vinorelbine, fulvestrant, toremifene, protein-bound paclitaxel, testolactone, bevacizumab, cytochalasin B, gramicidin D, ethidium bromide, emetine, mitomycin, etoposide, tenoposide, vincristine, vinblastine, colchicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, dactinomycin, bleomycin, mithramycin, anthramycin, calicheamicins, duocarmycins, alkylphosphocholines, topoisomerase I inhibitors, taxoids or suramins.

**12.** Use according to claim 7, 8, 9 or 10, wherein the agent or the antibody is able to downregulate the expression of the PTK7 polypeptide and / or wherein the agent or antibody is able to decrease the activity of the PTK7 polypeptide.

**13.** A method of screening for an agent for the treatment and prophylaxis of angiogenesis, invasion and metastasis of triple negative breast cancer cells or basal-like breast cancer cells, wherein said agent interacts with or modulates the expression or activity of the PTK7 polypeptide, said method comprising:
(a) contacting the PTK7 polypeptide with a candidate agent; and (b) determining whether or not the candidate agent interacts with said PTK7 polypeptide.

**14.** Method according to claim 13 further comprising the steps c) and d):
c) comparing the expression or activity of the PTK7 polypeptide in the presence of the candidate agent with the expression or activity of the PTK7 polypeptide in the absence of the candidate agent or in the presence of a control agent; and d) determining whether the candidate agent causes a change in the expression or activity of the PTK7 polypeptide .


SEQUENCE LISTING

<110> Max-Planck-Gesellschaft zur Förderung der Wissenschaften
e.V.

<120> PTK-7 protein involved in breast cancer

<130> ULA-P03107EP

<140> EP111111
<141> 2010-04-26

<160> 7

<170> PatentIn version 3.3

<210> 1
<211> 1070
<212> PRT
<213> Homo sapiens

<400> 1

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro

180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly

450                 455                 460

Thr Leu Arg Ile Asn Ser Val Glu Val Tyr Asp Gly Thr Trp Tyr Arg
465                 470         .       475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly
        515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Gly Lys Asp Arg Ile Leu Asp Pro Thr Lys Leu Gly Pro Arg
625                 630                 635                 640

Met His Ile Phe Gln Asn Gly Ser Leu Val Ile His Asp Val Ala Pro
                645                 650                 655

Glu Asp Ser Gly Arg Tyr Thr Cys Ile Ala Gly Asn Ser Cys Asn Ile
            660                 665                 670

Lys His Thr Glu Ala Pro Leu Tyr Val Val Asp Lys Pro Val Pro Glu
        675                 680                 685

Glu Ser Glu Gly Pro Gly Ser Pro Pro Pro Tyr Lys Met Ile Gln Thr
    690                 695                 700

Ile Gly Leu Ser Val Gly Ala Ala Val Ala Tyr Ile Ile Ala Val Leu
705                 710                 715                 720

Gly Leu Met Phe Tyr Cys Lys Lys Arg Cys Lys Ala Lys Arg Leu Gln

725                         730                         735

Lys Gln Pro Glu Gly Glu Glu Pro Glu Met Glu Cys Leu Asn Gly Gly
            740                 745                 750

Pro Leu Gln Asn Gly Gln Pro Ser Ala Glu Ile Gln Glu Glu Val Ala
        755                 760                 765

Leu Thr Ser Leu Gly Ser Gly Pro Ala Ala Thr Asn Lys Arg His Ser
    770                 775                 780

Thr Ser Asp Lys Met His Phe Pro Arg Ser Ser Leu Gln Pro Ile Thr
785                 790                 795                 800

Thr Leu Gly Lys Ser Glu Phe Gly Glu Val Phe Leu Ala Lys Ala Gln
                805                 810                 815

Gly Leu Glu Glu Gly Val Ala Glu Thr Leu Val Leu Val Lys Ser Leu
            820                 825                 830

Gln Ser Lys Asp Glu Gln Gln Gln Leu Asp Phe Arg Arg Glu Leu Glu
        835                 840                 845

Met Phe Gly Lys Leu Asn His Ala Asn Val Val Arg Leu Leu Gly Leu
    850                 855                 860

Cys Arg Glu Ala Glu Pro His Tyr Met Val Leu Glu Tyr Val Asp Leu
865                 870                 875                 880

Gly Asp Leu Lys Gln Phe Leu Arg Ile Ser Lys Ser Lys Asp Glu Lys
                885                 890                 895

Leu Lys Ser Gln Pro Leu Ser Thr Lys Gln Lys Val Ala Leu Cys Thr
            900                 905                 910

Gln Val Ala Leu Gly Met Glu His Leu Ser Asn Asn Arg Phe Val His
        915                 920                 925

Lys Asp Leu Ala Ala Arg Asn Cys Leu Val Ser Ala Gln Arg Gln Val
    930                 935                 940

Lys Val Ser Ala Leu Gly Leu Ser Lys Asp Val Tyr Asn Ser Glu Tyr
945                 950                 955                 960

Tyr His Phe Arg Gln Ala Trp Val Pro Leu Arg Trp Met Ser Pro Glu
                965                 970                 975

Ala Ile Leu Glu Gly Asp Phe Ser Thr Lys Ser Asp Val Trp Ala Phe
            980                 985                 990

Gly Val Leu Met Trp Glu Val Phe  Thr His Gly Glu Met  Pro His Gly

995                    1000                    1005

Gly Gln  Ala Asp Asp Glu Val  Leu Ala Asp Leu Gln  Ala Gly Lys
    1010                    1015                    1020

Ala Arg  Leu Pro Gln Pro Glu  Gly Cys Pro Ser Lys  Leu Tyr Arg
    1025                    1030                    1035

Leu Met  Gln Arg Cys Trp Ala  Leu Ser Pro Lys Asp  Arg Pro Ser
    1040                    1045                    1050

Phe Ser  Glu Ile Ala Ser Ala  Leu Gly Asp Ser Thr  Val Asp Ser
    1055                    1060                    1065

Lys Pro
    1070

<210>  2
<211>  1030
<212>  PRT
<213>  Homo sapiens

<400>  2

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Asp Gly Ser Ser Leu Pro Glu Trp Val Thr Asp Asn Ala
            500                 505                 510

Gly Thr Leu His Phe Ala Arg Val Thr Arg Asp Asp Ala Gly Asn Tyr
        515                 520                 525

Thr Cys Ile Ala Ser Asn Gly Pro Gln Gly Gln Ile Arg Ala His Val
    530                 535                 540

Gln Leu Thr Val Ala Val Phe Ile Thr Phe Lys Val Glu Pro Glu Arg
545                 550                 555                 560

Thr Thr Val Tyr Gln Gly His Thr Ala Leu Leu Gln Cys Glu Ala Gln
                565                 570                 575

Gly Asp Pro Lys Pro Leu Ile Gln Trp Lys Gly Lys Asp Arg Ile Leu
            580                 585                 590

Asp Pro Thr Lys Leu Gly Pro Arg Met His Ile Phe Gln Asn Gly Ser
        595                 600                 605

Leu Val Ile His Asp Val Ala Pro Glu Asp Ser Gly Arg Tyr Thr Cys
    610                 615                 620

Ile Ala Gly Asn Ser Cys Asn Ile Lys His Thr Glu Ala Pro Leu Tyr
625                 630                 635                 640

Val Val Asp Lys Pro Val Pro Glu Glu Ser Glu Gly Pro Gly Ser Pro
                645                 650                 655

Pro Pro Tyr Lys Met Ile Gln Thr Ile Gly Leu Ser Val Gly Ala Ala
            660                 665                 670

Val Ala Tyr Ile Ile Ala Val Leu Gly Leu Met Phe Tyr Cys Lys Lys
        675                 680                 685

Arg Cys Lys Ala Lys Arg Leu Gln Lys Gln Pro Glu Gly Glu Glu Pro
    690                 695                 700

Glu Met Glu Cys Leu Asn Gly Gly Pro Leu Gln Asn Gly Gln Pro Ser
705                 710                 715                 720

Ala Glu Ile Gln Glu Glu Val Ala Leu Thr Ser Leu Gly Ser Gly Pro
                725                 730                 735

Ala Ala Thr Asn Lys Arg His Ser Thr Ser Asp Lys Met His Phe Pro
            740                 745                 750

Arg Ser Ser Leu Gln Pro Ile Thr Thr Leu Gly Lys Ser Glu Phe Gly
        755                 760                 765

Glu Val Phe Leu Ala Lys Ala Gln Gly Leu Glu Glu Gly Val Ala Glu
    770                 775                 780

Thr Leu Val Leu Val Lys Ser Leu Gln Ser Lys Asp Glu Gln Gln Gln
785                 790                 795                 800

Leu Asp Phe Arg Arg Glu Leu Glu Met Phe Gly Lys Leu Asn His Ala
                805                 810                 815

Asn Val Val Arg Leu Leu Gly Leu Cys Arg Glu Ala Glu Pro His Tyr
            820                 825                 830

Met Val Leu Glu Tyr Val Asp Leu Gly Asp Leu Lys Gln Phe Leu Arg
        835                 840                 845

Ile Ser Lys Ser Lys Asp Glu Lys Leu Lys Ser Gln Pro Leu Ser Thr
    850                 855                 860

Lys Gln Lys Val Ala Leu Cys Thr Gln Val Ala Leu Gly Met Glu His
865                 870                 875                 880

Leu Ser Asn Asn Arg Phe Val His Lys Asp Leu Ala Ala Arg Asn Cys
                885                 890                 895

Leu Val Ser Ala Gln Arg Gln Val Lys Val Ser Ala Leu Gly Leu Ser
            900                 905                 910

Lys Asp Val Tyr Asn Ser Glu Tyr Tyr His Phe Arg Gln Ala Trp Val
        915                 920                 925

Pro Leu Arg Trp Met Ser Pro Glu Ala Ile Leu Glu Gly Asp Phe Ser
    930                 935                 940

Thr Lys Ser Asp Val Trp Ala Phe Gly Val Leu Met Trp Glu Val Phe
945                 950                 955                 960

Thr His Gly Glu Met Pro His Gly Gly Gln Ala Asp Asp Glu Val Leu
                965                 970                 975

Ala Asp Leu Gln Ala Gly Lys Ala Arg Leu Pro Gln Pro Glu Gly Cys
            980                 985                 990

Pro Ser Lys Leu Tyr Arg Leu Met Gln Arg Cys Trp Ala Leu Ser Pro
        995                 1000                1005

Lys Asp Arg Pro Ser Phe Ser Glu Ile Ala Ser Ala Leu Gly Asp
    1010                1015                1020

Ser Thr Val Asp Ser Lys Pro
    1025                1030

<210> 3
<211> 940
<212> PRT
<213> Homo sapiens

<400> 3

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Asn Gly Ser Ser Leu Pro Glu
                405                 410                 415

Trp Val Thr Asp Asn Ala Gly Thr Leu His Phe Ala Arg Val Thr Arg
            420                 425                 430

Asp Asp Ala Gly Asn Tyr Thr Cys Ile Ala Ser Asn Gly Pro Gln Gly
        435                 440                 445

Gln Ile Arg Ala His Val Gln Leu Thr Val Ala Val Phe Ile Thr Phe
    450                 455                 460

Lys Val Glu Pro Glu Arg Thr Thr Val Tyr Gln Gly His Thr Ala Leu
465                 470                 475                 480

Leu Gln Cys Glu Ala Gln Gly Asp Pro Lys Pro Leu Ile Gln Trp Lys
                485                 490                 495

Gly Lys Asp Arg Ile Leu Asp Pro Thr Lys Leu Gly Pro Arg Met His
            500                 505                 510

Ile Phe Gln Asn Gly Ser Leu Val Ile His Asp Val Ala Pro Glu Asp
        515                 520                 525

Ser Gly Arg Tyr Thr Cys Ile Ala Gly Asn Ser Cys Asn Ile Lys His
    530                 535                 540

Thr Glu Ala Pro Leu Tyr Val Val Asp Lys Pro Val Pro Glu Glu Ser
545                 550                 555                 560

Glu Gly Pro Gly Ser Pro Pro Pro Tyr Lys Met Ile Gln Thr Ile Gly
                565                 570                 575

Leu Ser Val Gly Ala Ala Val Ala Tyr Ile Ile Ala Val Leu Gly Leu
            580                 585                 590

Met Phe Tyr Cys Lys Lys Arg Cys Lys Ala Lys Arg Leu Gln Lys Gln
        595                 600                 605

Pro Glu Gly Glu Glu Pro Glu Met Glu Cys Leu Asn Gly Gly Pro Leu
    610                 615                 620

Gln Asn Gly Gln Pro Ser Ala Glu Ile Gln Glu Glu Val Ala Leu Thr
625                 630                 635                 640

Ser Leu Gly Ser Gly Pro Ala Ala Thr Asn Lys Arg His Ser Thr Ser
                645                 650                 655

Asp Lys Met His Phe Pro Arg Ser Ser Leu Gln Pro Ile Thr Thr Leu
            660                 665                 670

Gly Lys Ser Glu Phe Gly Glu Val Phe Leu Ala Lys Ala Gln Gly Leu
        675                 680                 685

Glu Glu Gly Val Ala Glu Thr Leu Val Leu Val Lys Ser Leu Gln Ser
    690                 695                 700

Lys Asp Glu Gln Gln Gln Leu Asp Phe Arg Arg Glu Leu Glu Met Phe
705                 710                 715                 720

Gly Lys Leu Asn His Ala Asn Val Val Arg Leu Leu Gly Leu Cys Arg
                725                 730                 735

Glu Ala Glu Pro His Tyr Met Val Leu Glu Tyr Val Asp Leu Gly Asp
            740                 745                 750

Leu Lys Gln Phe Leu Arg Ile Ser Lys Ser Lys Asp Glu Lys Leu Lys
        755                 760                 765

Ser Gln Pro Leu Ser Thr Lys Gln Lys Val Ala Leu Cys Thr Gln Val
    770                 775                 780

Ala Leu Gly Met Glu His Leu Ser Asn Asn Arg Phe Val His Lys Asp
785                 790                 795                 800

Leu Ala Ala Arg Asn Cys Leu Val Ser Ala Gln Arg Gln Val Lys Val
                805                 810                 815

Ser Ala Leu Gly Leu Ser Lys Asp Val Tyr Asn Ser Glu Tyr Tyr His
            820                 825                 830

Phe Arg Gln Ala Trp Val Pro Leu Arg Trp Met Ser Pro Glu Ala Ile
        835                 840                 845

Leu Glu Gly Asp Phe Ser Thr Lys Ser Asp Val Trp Ala Phe Gly Val
    850                 855                 860

Leu Met Trp Glu Val Phe Thr His Gly Glu Met Pro His Gly Gly Gln
865                 870                 875                 880

Ala Asp Asp Glu Val Leu Ala Asp Leu Gln Ala Gly Lys Ala Arg Leu
                885                 890                 895

Pro Gln Pro Glu Gly Cys Pro Ser Lys Leu Tyr Arg Leu Met Gln Arg
            900                 905                 910

Cys Trp Ala Leu Ser Pro Lys Asp Arg Pro Ser Phe Ser Glu Ile Ala
        915                 920                 925

Ser Ala Leu Gly Asp Ser Thr Val Asp Ser Lys Pro
    930                 935                 940

<210>  4
<211>  1014
<212>  PRT
<213>  Homo sapiens

<400>  4

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly
        515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Asp Lys Pro Val Pro Glu Glu Ser Glu Gly Pro Gly Ser Pro
625                 630                 635                 640

Pro Pro Tyr Lys Met Ile Gln Thr Ile Gly Leu Ser Val Gly Ala Ala
                645                 650                 655

Val Ala Tyr Ile Ile Ala Val Leu Gly Leu Met Phe Tyr Cys Lys Lys
            660                 665                 670

Arg Cys Lys Ala Lys Arg Leu Gln Lys Gln Pro Glu Gly Glu Glu Pro
        675                 680                 685

Glu Met Glu Cys Leu Asn Gly Gly Pro Leu Gln Asn Gly Gln Pro Ser
    690                 695                 700

Ala Glu Ile Gln Glu Glu Val Ala Leu Thr Ser Leu Gly Ser Gly Pro
705                 710                 715                 720

Ala Ala Thr Asn Lys Arg His Ser Thr Ser Asp Lys Met His Phe Pro
                725                 730                 735

Arg Ser Ser Leu Gln Pro Ile Thr Thr Leu Gly Lys Ser Glu Phe Gly
            740                 745                 750

Glu Val Phe Leu Ala Lys Ala Gln Gly Leu Glu Glu Gly Val Ala Glu
        755                 760                 765

Thr Leu Val Leu Val Lys Ser Leu Gln Ser Lys Asp Glu Gln Gln Gln
    770                 775                 780

Leu Asp Phe Arg Arg Glu Leu Glu Met Phe Gly Lys Leu Asn His Ala
785                 790                 795                 800

Asn Val Val Arg Leu Leu Gly Leu Cys Arg Glu Ala Glu Pro His Tyr
                805                 810                 815

Met Val Leu Glu Tyr Val Asp Leu Gly Asp Leu Lys Gln Phe Leu Arg
            820                 825                 830

Ile Ser Lys Ser Lys Asp Glu Lys Leu Lys Ser Gln Pro Leu Ser Thr
        835                 840                 845

Lys Gln Lys Val Ala Leu Cys Thr Gln Val Ala Leu Gly Met Glu His
    850                 855                 860

Leu Ser Asn Asn Arg Phe Val His Lys Asp Leu Ala Ala Arg Asn Cys
865                 870                 875                 880

Leu Val Ser Ala Gln Arg Gln Val Lys Val Ser Ala Leu Gly Leu Ser
                885                 890                 895

Lys Asp Val Tyr Asn Ser Glu Tyr Tyr His Phe Arg Gln Ala Trp Val
            900                 905                 910

Pro Leu Arg Trp Met Ser Pro Glu Ala Ile Leu Glu Gly Asp Phe Ser
        915                 920                 925

Thr Lys Ser Asp Val Trp Ala Phe Gly Val Leu Met Trp Glu Val Phe
    930                 935                 940

Thr His Gly Glu Met Pro His Gly Gly Gln Ala Asp Asp Glu Val Leu
945                 950                 955                 960

Ala Asp Leu Gln Ala Gly Lys Ala Arg Leu Pro Gln Pro Glu Gly Cys
                965                 970                 975

Pro Ser Lys Leu Tyr Arg Leu Met Gln Arg Cys Trp Ala Leu Ser Pro
            980                 985                 990

Lys Asp Arg Pro Ser Phe Ser Glu  Ile Ala Ser Ala Leu  Gly Asp Ser
        995                 1000                1005

Thr Val  Asp Ser Lys Pro
    1010

<210>  5
<211>  64
<212>  DNA
<213>  Homo sapiens

<400>  5
gatccccgga tgtgtacaac agtgagttca agagactcac tgttgtacac atcctttttg      60

gaaa                                                                    64

<210>  6
<211>  19
<212>  DNA
<213>  Homo sapiens

<400>  6
ggatgtgtac aacagtgag                                                    19

<210>  7
<211>  736
<212>  PRT
<213>  Homo sapiens

<400>  7

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu

245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly

515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Gly Lys Asp Arg Ile Leu Asp Pro Thr Lys Leu Gly Pro Arg
625                 630                 635                 640

Met His Ile Phe Gln Asn Gly Ser Leu Val Ile His Asp Val Ala Pro
                645                 650                 655

Glu Asp Ser Gly Arg Tyr Thr Cys Ile Ala Gly Asn Ser Cys Asn Ile
            660                 665                 670

Lys His Thr Glu Ala Pro Leu Tyr Val Val Asp Lys Pro Val Pro Glu
        675                 680                 685

Glu Ser Glu Gly Pro Gly Ser Pro Pro Pro Tyr Lys Met Ile Gln Thr
    690                 695                 700

Ile Gly Leu Ser Val Gly Ala Ala Val Ala Tyr Ile Ile Ala Val Leu
705                 710                 715                 720

Gly Leu Met Phe Tyr Cys Lys Lys Arg Cys Lys Ala Lys Arg Leu Gln
                725                 730                 735

Sequence Listings

Seq. ID. No. 1
page 7: Amino acid sequences of four splice variants of PTK7
Genebank Accession: NP_002812.2 (PTK7-1)
MGAARGSPAR PRRLPLLSVL LLPLLGGTQT AIVFIKQPSS QDALQGRRAL LRCEVEAPGP
VHVYWLLDGA PVQDTERRFA QGSSLSFAAV DRLQDSGTFQ CVARDDVTGE EARSANASFN
IKWIEAGPVV LKHPASEAEI QPQTQVTLRC HIDGHPRPTY QWFRDGTPLS DGQSNHTVSS
KERNLTLRPA GPEHSGLYSC CAHSAFGQAC SSQNFTLSIA DESFARVVLA PQDVVVARYE
EAMFHCQFSA QPPPSLQWLF EDETPITNRS RPPHLRRATV FANGSLLLTQ VRPRNAGIYR
CIGQGQRGPP IILEATLHLA EIEDMPLFEP RVFTAGSEER VTCLPPKGLP EPSVWWEHAG
VRLPTHGRVY QKGHELVLAN IAESDAGVYT CHAANLAGQR RQDVNITVAT VPSWLKKPQD
SQLEEGKPGY LDCLTQATPK PTVVWYRNQM LISEDSRFEV FKNGTLRINS VEVYDGTWYR
CMSSTPAGSI EAQARVQVLE KLKFTPPPQP QQCMEFDKEA TVPCSATGRE KPTIKWERAD
GSSLPEWVTD NAGTLHFARV TRDDAGNYTC IASNGPQGQI RAHVQLTVAV FITFKVEPER
TTVYQGHTAL LQCEAQGDPK PLIQWKGKDR ILDPTKLGPR MHIFQNGSLV IHDVAPEDSG
RYTCIAGNSC NIKHTEAPLY VVDKPVPEES EGPGSPPPYK MIQTIGLSVG AAVAYIIAVL
GLMFYCKKRC KAKRLQKQPE GEEPEMECLN GGPLQNGQPS AEIQEEVALT SLGSGPAATN
KRHSTSDKMH FPRSSLQPIT TLGKSEFGEV FLAKAQGLEE GVAETLVLVK SLQSKDEQQQ
LDFRRELEMF GKLNHANVVR LLGLCREAEP HYMVLEYVDL GDLKQFLRIS KSKDEKLKSQ
PLSTKQKVAL CTQVALGMEH LSNNRFVHKD LAARNCLVSA QRQVKVSALG LSKDVYNSEY
YHFRQAWVPL RWMSPEAILE GDFSTKSDVW AFGVLMWEVF THGEMPHGGQ ADDEVLADLQ
AGKARLPQPE GCPSKLYRLM QRCWALSPKD RPSFSEIASA LGDSTVDSKP

Seq. ID. No. 2
Genebank Accession: NP_690619.1 (PTK7-2)
MGAARGSPAR PRRLPLLSVL LLPLLGGTQT AIVFIKQPSS QDALQGRRAL LRCEVEAPGP
VHVYWLLDGA PVQDTERRFA QGSSLSFAAV DRLQDSGTFQ CVARDDVTGE EARSANASFN
IKWIEAGPVV LKHPASEAEI QPQTQVTLRC HIDGHPRPTY QWFRDGTPLS DGQSNHTVSS
KERNLTLRPA GPEHSGLYSC CAHSAFGQAC SSQNFTLSIA DESFARVVLA PQDVVVARYE
EAMFHCQFSA QPPPSLQWLF EDETPITNRS RPPHLRRATV FANGSLLLTQ VRPRNAGIYR
CIGQGQRGPP IILEATLHLA EIEDMPLFEP RVFTAGSEER VTCLPPKGLP EPSVWWEHAG
VRLPTHGRVY QKGHELVLAN IAESDAGVYT CHAANLAGQR RQDVNITVAT VPSWLKKPQD
SQLEEGKPGY LDCLTQATPK PTVVWYRNQM LISEDSRFEV FKNGTLRINS VEVYDGTWYR
CMSSTPAGSI EAQARVQVLD GSSLPEWVTD NAGTLHFARV TRDDAGNYTC IASNGPQGQI
RAHVQLTVAV FITFKVEPER TTVYQGHTAL LQCEAQGDPK PLIQWKGKDR ILDPTKLGPR
MHIFQNGSLV IHDVAPEDSG RYTCIAGNSC NIKHTEAPLY VVDKPVPEES EGPGSPPPYK
MIQTIGLSVG AAVAYIIAVL GLMFYCKKRC KAKRLQKQPE GEEPEMECLN GGPLQNGQPS
AEIQEEVALT SLGSGPAATN KRHSTSDKMH FPRSSLQPIT TLGKSEFGEV FLAKAQGLEE
GVAETLVLVK SLQSKDEQQQ LDFRRELEMF GKLNHANVVR LLGLCREAEP HYMVLEYVDL
GDLKQFLRIS KSKDEKLKSQ PLSTKQKVAL CTQVALGMEH LSNNRFVHKD LAARNCLVSA
QRQVKVSALG LSKDVYNSEY YHFRQAWVPL RWMSPEAILE GDFSTKSDVW AFGVLMWEVF
THGEMPHGGQ ADDEVLADLQ AGKARLPQPE GCPSKLYRLM QRCWALSPKD RPSFSEIASA
LGDSTVDSKP

Seq. ID. No. 3
Genebank Accession: NP_690620.1 (PTK7-3)
MGAARGSPAR PRRLPLLSVL LLPLLGGTQT AIVFIKQPSS QDALQGRRAL LRCEVEAPGP
VHVYWLLDGA PVQDTERRFA QGSSLSFAAV DRLQDSGTFQ CVARDDVTGE EARSANASFN
IKWIEAGPVV LKHPASEAEI QPQTQVTLRC HIDGHPRPTY QWFRDGTPLS DGQSNHTVSS
KERNLTLRPA GPEHSGLYSC CAHSAFGQAC SSQNFTLSIA DESFARVVLA PQDVVVARYE
EAMFHCQFSA QPPPSLQWLF EDETPITNRS RPPHLRRATV FANGSLLLTQ VRPRNAGIYR
CIGQGQRGPP IILEATLHLA EIEDMPLFEP RVFTAGSEER VTCLPPKGLP EPSVWWEHAG
VRLPTHGRVY QKGHELVLAN IAESDAGVYT CHAANLAGQR RQDVNITVAN GSSLPEWVTD

NAGTLHFARV TRDDAGNYTC IASNGPQGQI RAHVQLTVAV FITFKVEPER TTVYQGHTAL
LQCEAQGDPK PLIQWKGKDR ILDPTKLGPR MHIFQNGSLV IHDVAPEDSG RYTCIAGNSC
NIKHTEAPLY VVDKPVPEES EGPGSPPPYK MIQTIGLSVG AAVAYIIAVL GLMFYCKKRC
KAKRLQKQPE GEEPEMECLN GGPLQNGQPS AEIQEEVALT SLGSGPAATN KRHSTSDKMH
FPRSSLQPIT TLGKSEFGEV FLAKAQGLEE GVAETLVLVK SLQSKDEQQQ LDFRRELEMF
GKLNHANVVR LLGLCREAEP HYMVLEYVDL GDLKQFLRIS KSKDEKLKSQ PLSTKQKVAL
CTQVALGMEH LSNNRFVHKD LAARNCLVSA QRQVKVSALG LSKDVYNSEY YHFRQAWVPL
RWMSPEAILE GDFSTKSDVW AFGVLMWEVF THGEMPHGGQ ADDEVLADLQ AGKARLPQPE
GCPSKLYRLM QRCWALSPKD RPSFSEIASA LGDSTVDSKP

Seq. ID. No. 4
Genebank Accession: NP_690621.1 (PTK7-4)
MGAARGSPAR PRRLPLLSVL LLPLLGGTQT AIVFIKQPSS QDALQGRRAL LRCEVEAPGP
VHVYWLLDGA PVQDTERRFA QGSSLSFAAV DRLQDSGTFQ CVARDDVTGE EARSANASFN
IKWIEAGPVV LKHPASEAEI QPQTQVTLRC HIDGHPRPTY QWFRDGTPLS DGQSNHTVSS
KERNLTLRPA GPEHSGLYSC CAHSAFGQAC SSQNFTLSIA DESFARVVLA PQDVVVARYE
EAMFHCQFSA QPPPSLQWLF EDETPITNRS RPPHLRRATV FANGSLLLTQ VRPRNAGIYR
CIGQGQRGPP IILEATLHLA EIEDMPLFEP RVFTAGSEER VTCLPPKGLP EPSVWWEHAG
VRLPTHGRVY QKGHELVLAN IAESDAGVYT CHAANLAGQR RQDVNITVAT VPSWLKKPQD
SQLEEGKPGY LDCLTQATPK PTVVWYRNQM LISEDSRFEV FKNGTLRINS VEVYDGTWYR
CMSSTPAGSI EAQARVQVLE KLKFTPPPQP QQCMEFDKEA TVPCSATGRE KPTIKWERAD
GSSLPEWVTD NAGTLHFARV TRDDAGNYTC IASNGPQGQI RAHVQLTVAV FITFKVEPER
TTVYQGHTAL LQCEAQGDPK PLIQWKDKPV PEESEGPGSP PPYKMIQTIG LSVGAAVAYI
IAVLGLMFYC KKRCKAKRLQ KQPEGEEPEM ECLNGGPLQN GQPSAEIQEE VALTSLGSGP
AATNKRHSTS DKMHFPRSSL QPITTLGKSE FGEVFLAKAQ GLEEGVAETL VLVKSLQSKD
EQQQLDFRRE LEMFGKLNHA NVVRLLGLCR EAEPHYMVLE YVDLGDLKQF LRISKSKDEK
LKSQPLSTKQ KVALCTQVAL GMEHLSNNRF VHKDLAARNC LVSAQRQVKV SALGLSKDVY
NSEYYHFRQA WVPLRWMSPE AILEGDFSTK SDVWAFGVLM WEVFTHGEMP HGGQADDEVL
ADLQAGKARL PQPEGCPSKL YRLMQRCWAL SPKDRPSFSE IASALGDSTV DSKP

Seq. ID. No. 5
page 17, figure 6: siRNA sequence
siRNA oligo for cloning into the pSUPERretro expression vector

GATCCCCGGATGTGTACAACAGTGAGttcaagagaCTCACTGTTGTACACATCCTTTTTGGAAA

Seq. ID. No. 6
targeted sequence
GGATGTGTACAACAGTGAG

Seq. ID. No. 7
page 17, figure 7: Amino acid sequence of dominant negative PTK7 (PTK7ΔCT)
MGAARGSPARPRRLPLLSVLLLPLLGGTQTAIVFIKQPSSQDALQGRRALLRCEVEAPGPVHVYWLLDGAPVQDT
ERRFAQGSSLSFAAVDRLQDSGTFQCVARDDVTGEEARSANASFNIKWIEAGPVVLKHPASEAEIQPQTQVTLRC
HIDGHPRPTYQWFRDGTPLSDGQSNHTVSSKERNLTLRPAGPEHSGLYSCCAHSAFGQACSSQNFTLSIADESFA
RVVLAPQDVVVARYEEAMFHCQFSAQPPPSLQWLFEDETPITNRSRPPHLRRATVFANGSLLLTQVRPRNAGIYR
CIGQGQRGPPIILEATLHLAEIEDMPLFEPRVFTAGSEERVTCLPPKGLPEPSVWWEHAGVRLPTHGRVYQKGHE
LVLANIAESDAGVYTCHAANLAGQRRQDVNITVATVPSWLKKPQDSQLEEGKPGYLDCLTQATPKPTVVWYRNQM
LISEDSRFEVFKNGTLRINSVEVYDGTWYRCMSSTPAGSIEAQARVQVLEKLKFTPPPQPQQCMEFDKEATVPCS
ATGREKPTIKWERADGSSLPEWVTDNAGTLHFARVTRDDAGNYTCIASNGPQGQIRAHVQLTVAVFITFKVEPER
TTVYQGHTALLQCEAQGDPKPLIQWKGKDRILDPTKLGPRMHIFQNGSLVIHDVAPEDSGRYTCIAGNSCNIKHT
EAPLYVVDKPVPEESEGPGSPPPYKMIQTIGLSVGAAVAYIIAVLGLMFYCKKRCKAKRLQ

SEQUENCE LISTING

<110>  Max-Planck-Gesellschaft zur Förderung der Wissenschaften
       e.V.

<120>  PTK-7 protein involved in breast cancer

<130>  ULA-P03107EP

<140>  EP111111
<141>  2010-04-26

<160>  11

<170>  PatentIn version 3.3

<210>  1
<211>  1070
<212>  PRT
<213>  Homo sapiens

<400>  1

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro

180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly

450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly
        515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Gly Lys Asp Arg Ile Leu Asp Pro Thr Lys Leu Gly Pro Arg
625                 630                 635                 640

Met His Ile Phe Gln Asn Gly Ser Leu Val Ile His Asp Val Ala Pro
                645                 650                 655

Glu Asp Ser Gly Arg Tyr Thr Cys Ile Ala Gly Asn Ser Cys Asn Ile
            660                 665                 670

Lys His Thr Glu Ala Pro Leu Tyr Val Val Asp Lys Pro Val Pro Glu
        675                 680                 685

Glu Ser Glu Gly Pro Gly Ser Pro Pro Pro Tyr Lys Met Ile Gln Thr
    690                 695                 700

Ile Gly Leu Ser Val Gly Ala Ala Val Ala Tyr Ile Ile Ala Val Leu
705                 710                 715                 720

Gly Leu Met Phe Tyr Cys Lys Lys Arg Cys Lys Ala Lys Arg Leu Gln

725                 730                 735

Lys Gln Pro Glu Gly Glu Glu Pro Glu Met Glu Cys Leu Asn Gly Gly
            740                 745                 750

Pro Leu Gln Asn Gly Gln Pro Ser Ala Glu Ile Gln Glu Glu Val Ala
        755                 760                 765

Leu Thr Ser Leu Gly Ser Gly Pro Ala Ala Thr Asn Lys Arg His Ser
    770                 775                 780

Thr Ser Asp Lys Met His Phe Pro Arg Ser Ser Leu Gln Pro Ile Thr
785                 790                 795                 800

Thr Leu Gly Lys Ser Glu Phe Gly Glu Val Phe Leu Ala Lys Ala Gln
                805                 810                 815

Gly Leu Glu Glu Gly Val Ala Glu Thr Leu Val Leu Val Lys Ser Leu
            820                 825                 830

Gln Ser Lys Asp Glu Gln Gln Gln Leu Asp Phe Arg Arg Glu Leu Glu
        835                 840                 845

Met Phe Gly Lys Leu Asn His Ala Asn Val Val Arg Leu Leu Gly Leu
    850                 855                 860

Cys Arg Glu Ala Glu Pro His Tyr Met Val Leu Glu Tyr Val Asp Leu
865                 870                 875                 880

Gly Asp Leu Lys Gln Phe Leu Arg Ile Ser Lys Ser Lys Asp Glu Lys
                885                 890                 895

Leu Lys Ser Gln Pro Leu Ser Thr Lys Gln Lys Val Ala Leu Cys Thr
            900                 905                 910

Gln Val Ala Leu Gly Met Glu His Leu Ser Asn Asn Arg Phe Val His
        915                 920                 925

Lys Asp Leu Ala Ala Arg Asn Cys Leu Val Ser Ala Gln Arg Gln Val
    930                 935                 940

Lys Val Ser Ala Leu Gly Leu Ser Lys Asp Val Tyr Asn Ser Glu Tyr
945                 950                 955                 960

Tyr His Phe Arg Gln Ala Trp Val Pro Leu Arg Trp Met Ser Pro Glu
                965                 970                 975

Ala Ile Leu Glu Gly Asp Phe Ser Thr Lys Ser Asp Val Trp Ala Phe
            980                 985                 990

Gly Val Leu Met Trp Glu Val Phe  Thr His Gly Glu Met  Pro His Gly

995                 1000                1005

Gly Gln  Ala Asp Asp Glu Val  Leu Ala Asp Leu Gln  Ala Gly Lys
    1010                 1015                 1020

Ala Arg  Leu Pro Gln Pro Glu  Gly Cys Pro Ser Lys  Leu Tyr Arg
    1025                 1030                 1035

Leu Met  Gln Arg Cys Trp Ala  Leu Ser Pro Lys Asp  Arg Pro Ser
    1040                 1045                 1050

Phe Ser  Glu Ile Ala Ser Ala  Leu Gly Asp Ser Thr  Val Asp Ser
    1055                 1060                 1065

Lys Pro
    1070

<210>  2
<211>  1030
<212>  PRT
<213>  Homo sapiens

<400>  2

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Asp Gly Ser Ser Leu Pro Glu Trp Val Thr Asp Asn Ala
            500                 505                 510

Gly Thr Leu His Phe Ala Arg Val Thr Arg Asp Asp Ala Gly Asn Tyr
        515                 520                 525

Thr Cys Ile Ala Ser Asn Gly Pro Gln Gly Gln Ile Arg Ala His Val
    530                 535                 540

Gln Leu Thr Val Ala Val Phe Ile Thr Phe Lys Val Glu Pro Glu Arg
545                 550                 555                 560

Thr Thr Val Tyr Gln Gly His Thr Ala Leu Leu Gln Cys Glu Ala Gln
                565                 570                 575

Gly Asp Pro Lys Pro Leu Ile Gln Trp Lys Gly Lys Asp Arg Ile Leu
            580                 585                 590

Asp Pro Thr Lys Leu Gly Pro Arg Met His Ile Phe Gln Asn Gly Ser
        595                 600                 605

Leu Val Ile His Asp Val Ala Pro Glu Asp Ser Gly Arg Tyr Thr Cys
    610                 615                 620

Ile Ala Gly Asn Ser Cys Asn Ile Lys His Thr Glu Ala Pro Leu Tyr
625                 630                 635                 640

Val Val Asp Lys Pro Val Pro Glu Glu Ser Glu Gly Pro Gly Ser Pro
                645                 650                 655

Pro Pro Tyr Lys Met Ile Gln Thr Ile Gly Leu Ser Val Gly Ala Ala
            660                 665                 670

Val Ala Tyr Ile Ile Ala Val Leu Gly Leu Met Phe Tyr Cys Lys Lys
        675                 680                 685

Arg Cys Lys Ala Lys Arg Leu Gln Lys Gln Pro Glu Gly Glu Glu Pro
    690                 695                 700

Glu Met Glu Cys Leu Asn Gly Gly Pro Leu Gln Asn Gly Gln Pro Ser
705                 710                 715                 720

Ala Glu Ile Gln Glu Glu Val Ala Leu Thr Ser Leu Gly Ser Gly Pro
                725                 730                 735

Ala Ala Thr Asn Lys Arg His Ser Thr Ser Asp Lys Met His Phe Pro
            740                 745                 750

Arg Ser Ser Leu Gln Pro Ile Thr Thr Leu Gly Lys Ser Glu Phe Gly
        755                 760                 765

Glu Val Phe Leu Ala Lys Ala Gln Gly Leu Glu Glu Gly Val Ala Glu
    770                 775                 780

Thr Leu Val Leu Val Lys Ser Leu Gln Ser Lys Asp Glu Gln Gln Gln
785                 790                 795                 800

Leu Asp Phe Arg Arg Glu Leu Glu Met Phe Gly Lys Leu Asn His Ala
                805                 810                 815

Asn Val Val Arg Leu Leu Gly Leu Cys Arg Glu Ala Glu Pro His Tyr
            820                 825                 830

Met Val Leu Glu Tyr Val Asp Leu Gly Asp Leu Lys Gln Phe Leu Arg
        835                 840                 845

Ile Ser Lys Ser Lys Asp Glu Lys Leu Lys Ser Gln Pro Leu Ser Thr
    850                 855                 860

Lys Gln Lys Val Ala Leu Cys Thr Gln Val Ala Leu Gly Met Glu His
865                 870                 875                 880

Leu Ser Asn Asn Arg Phe Val His Lys Asp Leu Ala Ala Arg Asn Cys
                885                 890                 895

Leu Val Ser Ala Gln Arg Gln Val Lys Val Ser Ala Leu Gly Leu Ser
            900                 905                 910

Lys Asp Val Tyr Asn Ser Glu Tyr Tyr His Phe Arg Gln Ala Trp Val
        915                 920                 925

Pro Leu Arg Trp Met Ser Pro Glu Ala Ile Leu Glu Gly Asp Phe Ser
    930                 935                 940

Thr Lys Ser Asp Val Trp Ala Phe Gly Val Leu Met Trp Glu Val Phe
945                 950                 955                 960

Thr His Gly Glu Met Pro His Gly Gly Gln Ala Asp Asp Glu Val Leu
                965                 970                 975

Ala Asp Leu Gln Ala Gly Lys Ala Arg Leu Pro Gln Pro Glu Gly Cys
            980                 985                 990

Pro Ser Lys Leu Tyr Arg Leu Met  Gln Arg Cys Trp Ala  Leu Ser Pro
        995                 1000                 1005

Lys Asp  Arg Pro Ser Phe Ser  Glu Ile Ala Ser Ala  Leu Gly Asp
    1010                 1015                 1020

Ser Thr  Val Asp Ser Lys Pro
    1025                 1030

<210>  3
<211>  940
<212>  PRT
<213>  Homo sapiens

<400>  3

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Asn Gly Ser Ser Leu Pro Glu
                405                 410                 415

Trp Val Thr Asp Asn Ala Gly Thr Leu His Phe Ala Arg Val Thr Arg
            420                 425                 430

Asp Asp Ala Gly Asn Tyr Thr Cys Ile Ala Ser Asn Gly Pro Gln Gly
        435                 440                 445

Gln Ile Arg Ala His Val Gln Leu Thr Val Ala Val Phe Ile Thr Phe
    450                 455                 460

Lys Val Glu Pro Glu Arg Thr Thr Val Tyr Gln Gly His Thr Ala Leu
465                 470                 475                 480

Leu Gln Cys Glu Ala Gln Gly Asp Pro Lys Pro Leu Ile Gln Trp Lys
                485                 490                 495

Gly Lys Asp Arg Ile Leu Asp Pro Thr Lys Leu Gly Pro Arg Met His
            500                 505                 510

Ile Phe Gln Asn Gly Ser Leu Val Ile His Asp Val Ala Pro Glu Asp
        515                 520                 525

Ser Gly Arg Tyr Thr Cys Ile Ala Gly Asn Ser Cys Asn Ile Lys His
    530                 535                 540

Thr Glu Ala Pro Leu Tyr Val Val Asp Lys Pro Val Pro Glu Glu Ser
545                 550                 555                 560

Glu Gly Pro Gly Ser Pro Pro Pro Tyr Lys Met Ile Gln Thr Ile Gly
                565                 570                 575

Leu Ser Val Gly Ala Ala Val Ala Tyr Ile Ile Ala Val Leu Gly Leu
            580                 585                 590

Met Phe Tyr Cys Lys Lys Arg Cys Lys Ala Lys Arg Leu Gln Lys Gln
        595                 600                 605

Pro Glu Gly Glu Glu Pro Glu Met Glu Cys Leu Asn Gly Gly Pro Leu
    610                 615                 620

Gln Asn Gly Gln Pro Ser Ala Glu Ile Gln Glu Glu Val Ala Leu Thr
625                 630                 635                 640

Ser Leu Gly Ser Gly Pro Ala Ala Thr Asn Lys Arg His Ser Thr Ser
                645                 650                 655

Asp Lys Met His Phe Pro Arg Ser Ser Leu Gln Pro Ile Thr Thr Leu
            660                 665                 670

Gly Lys Ser Glu Phe Gly Glu Val Phe Leu Ala Lys Ala Gln Gly Leu
        675                 680                 685

Glu Glu Gly Val Ala Glu Thr Leu Val Leu Val Lys Ser Leu Gln Ser
    690                 695                 700

Lys Asp Glu Gln Gln Gln Leu Asp Phe Arg Arg Glu Leu Glu Met Phe
705                 710                 715                 720

Gly Lys Leu Asn His Ala Asn Val Val Arg Leu Leu Gly Leu Cys Arg
                725                 730                 735

Glu Ala Glu Pro His Tyr Met Val Leu Glu Tyr Val Asp Leu Gly Asp
            740                 745                 750

Leu Lys Gln Phe Leu Arg Ile Ser Lys Ser Lys Asp Glu Lys Leu Lys
        755                 760                 765

Ser Gln Pro Leu Ser Thr Lys Gln Lys Val Ala Leu Cys Thr Gln Val
    770                 775                 780

Ala Leu Gly Met Glu His Leu Ser Asn Asn Arg Phe Val His Lys Asp
785                 790                 795                 800

Leu Ala Ala Arg Asn Cys Leu Val Ser Ala Gln Arg Gln Val Lys Val
                805                 810                 815

Ser Ala Leu Gly Leu Ser Lys Asp Val Tyr Asn Ser Glu Tyr Tyr His
            820                 825                 830

Phe Arg Gln Ala Trp Val Pro Leu Arg Trp Met Ser Pro Glu Ala Ile
        835                 840                 845

Leu Glu Gly Asp Phe Ser Thr Lys Ser Asp Val Trp Ala Phe Gly Val
    850                 855                 860

Leu Met Trp Glu Val Phe Thr His Gly Glu Met Pro His Gly Gly Gln
865                 870                 875                 880

Ala Asp Asp Glu Val Leu Ala Asp Leu Gln Ala Gly Lys Ala Arg Leu
                885                 890                 895

Pro Gln Pro Glu Gly Cys Pro Ser Lys Leu Tyr Arg Leu Met Gln Arg
            900                 905                 910

Cys Trp Ala Leu Ser Pro Lys Asp Arg Pro Ser Phe Ser Glu Ile Ala
        915                 920                 925

Ser Ala Leu Gly Asp Ser Thr Val Asp Ser Lys Pro
    930                 935                 940

<210>  4
<211>  1014
<212>  PRT
<213>  Homo sapiens

<400>  4

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu
                245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly
        515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Asp Lys Pro Val Pro Glu Glu Ser Glu Gly Pro Gly Ser Pro
625                 630                 635                 640

Pro Pro Tyr Lys Met Ile Gln Thr Ile Gly Leu Ser Val Gly Ala Ala
                645                 650                 655

Val Ala Tyr Ile Ile Ala Val Leu Gly Leu Met Phe Tyr Cys Lys Lys
            660                 665                 670

Arg Cys Lys Ala Lys Arg Leu Gln Lys Gln Pro Glu Gly Glu Glu Pro
        675                 680                 685

Glu Met Glu Cys Leu Asn Gly Gly Pro Leu Gln Asn Gly Gln Pro Ser
    690                 695                 700

Ala Glu Ile Gln Glu Glu Val Ala Leu Thr Ser Leu Gly Ser Gly Pro
705                 710                 715                 720

Ala Ala Thr Asn Lys Arg His Ser Thr Ser Asp Lys Met His Phe Pro
                725                 730                 735

Arg Ser Ser Leu Gln Pro Ile Thr Thr Leu Gly Lys Ser Glu Phe Gly
            740                 745                 750

Glu Val Phe Leu Ala Lys Ala Gln Gly Leu Glu Glu Gly Val Ala Glu
        755                 760                 765

Thr Leu Val Leu Val Lys Ser Leu Gln Ser Lys Asp Glu Gln Gln Gln
    770                 775                 780

Leu Asp Phe Arg Arg Glu Leu Glu Met Phe Gly Lys Leu Asn His Ala
785                 790                 795                 800

Asn Val Val Arg Leu Leu Gly Leu Cys Arg Glu Ala Glu Pro His Tyr
                805                 810                 815

Met Val Leu Glu Tyr Val Asp Leu Gly Asp Leu Lys Gln Phe Leu Arg
            820                 825                 830

Ile Ser Lys Ser Lys Asp Glu Lys Leu Lys Ser Gln Pro Leu Ser Thr
        835                 840                 845

Lys Gln Lys Val Ala Leu Cys Thr Gln Val Ala Leu Gly Met Glu His
    850                 855                 860

Leu Ser Asn Asn Arg Phe Val His Lys Asp Leu Ala Ala Arg Asn Cys
865                 870                 875                 880

Leu Val Ser Ala Gln Arg Gln Val Lys Val Ser Ala Leu Gly Leu Ser
                885                 890                 895

Lys Asp Val Tyr Asn Ser Glu Tyr Tyr His Phe Arg Gln Ala Trp Val
            900                 905                 910

Pro Leu Arg Trp Met Ser Pro Glu Ala Ile Leu Glu Gly Asp Phe Ser
        915                 920                 925

Thr Lys Ser Asp Val Trp Ala Phe Gly Val Leu Met Trp Glu Val Phe
    930                 935                 940

Thr His Gly Glu Met Pro His Gly Gly Gln Ala Asp Asp Glu Val Leu
945                 950                 955                 960

Ala Asp Leu Gln Ala Gly Lys Ala Arg Leu Pro Gln Pro Glu Gly Cys
                965                 970                 975

Pro Ser Lys Leu Tyr Arg Leu Met Gln Arg Cys Trp Ala Leu Ser Pro
            980                 985                 990

Lys Asp Arg Pro Ser Phe Ser Glu  Ile Ala Ser Ala Leu  Gly Asp Ser
        995                 1000                 1005

Thr Val  Asp Ser Lys Pro
    1010

<210>  5
<211>  64
<212>  DNA
<213>  Homo sapiens

<400>  5
gatccccgga tgtgtacaac agtgagttca agagactcac tgttgtacac atcctttttg      60

gaaa                                                                    64

<210>  6
<211>  19
<212>  DNA
<213>  Homo sapiens

<400>  6
ggatgtgtac aacagtgag                                                    19

<210>  7
<211>  736
<212>  PRT
<213>  Homo sapiens

<400>  7

Met Gly Ala Ala Arg Gly Ser Pro Ala Arg Pro Arg Arg Leu Pro Leu
1               5                   10                  15

Leu Ser Val Leu Leu Leu Pro Leu Leu Gly Gly Thr Gln Thr Ala Ile
            20                  25                  30

Val Phe Ile Lys Gln Pro Ser Ser Gln Asp Ala Leu Gln Gly Arg Arg
        35                  40                  45

Ala Leu Leu Arg Cys Glu Val Glu Ala Pro Gly Pro Val His Val Tyr
    50                  55                  60

Trp Leu Leu Asp Gly Ala Pro Val Gln Asp Thr Glu Arg Arg Phe Ala
65                  70                  75                  80

Gln Gly Ser Ser Leu Ser Phe Ala Ala Val Asp Arg Leu Gln Asp Ser
                85                  90                  95

Gly Thr Phe Gln Cys Val Ala Arg Asp Asp Val Thr Gly Glu Glu Ala
            100                 105                 110

Arg Ser Ala Asn Ala Ser Phe Asn Ile Lys Trp Ile Glu Ala Gly Pro
        115                 120                 125

Val Val Leu Lys His Pro Ala Ser Glu Ala Glu Ile Gln Pro Gln Thr
    130                 135                 140

Gln Val Thr Leu Arg Cys His Ile Asp Gly His Pro Arg Pro Thr Tyr
145                 150                 155                 160

Gln Trp Phe Arg Asp Gly Thr Pro Leu Ser Asp Gly Gln Ser Asn His
                165                 170                 175

Thr Val Ser Ser Lys Glu Arg Asn Leu Thr Leu Arg Pro Ala Gly Pro
            180                 185                 190

Glu His Ser Gly Leu Tyr Ser Cys Cys Ala His Ser Ala Phe Gly Gln
        195                 200                 205

Ala Cys Ser Ser Gln Asn Phe Thr Leu Ser Ile Ala Asp Glu Ser Phe
    210                 215                 220

Ala Arg Val Val Leu Ala Pro Gln Asp Val Val Val Ala Arg Tyr Glu
225                 230                 235                 240

Glu Ala Met Phe His Cys Gln Phe Ser Ala Gln Pro Pro Pro Ser Leu

245                 250                 255

Gln Trp Leu Phe Glu Asp Glu Thr Pro Ile Thr Asn Arg Ser Arg Pro
            260                 265                 270

Pro His Leu Arg Arg Ala Thr Val Phe Ala Asn Gly Ser Leu Leu Leu
        275                 280                 285

Thr Gln Val Arg Pro Arg Asn Ala Gly Ile Tyr Arg Cys Ile Gly Gln
    290                 295                 300

Gly Gln Arg Gly Pro Pro Ile Ile Leu Glu Ala Thr Leu His Leu Ala
305                 310                 315                 320

Glu Ile Glu Asp Met Pro Leu Phe Glu Pro Arg Val Phe Thr Ala Gly
                325                 330                 335

Ser Glu Glu Arg Val Thr Cys Leu Pro Pro Lys Gly Leu Pro Glu Pro
            340                 345                 350

Ser Val Trp Trp Glu His Ala Gly Val Arg Leu Pro Thr His Gly Arg
        355                 360                 365

Val Tyr Gln Lys Gly His Glu Leu Val Leu Ala Asn Ile Ala Glu Ser
    370                 375                 380

Asp Ala Gly Val Tyr Thr Cys His Ala Ala Asn Leu Ala Gly Gln Arg
385                 390                 395                 400

Arg Gln Asp Val Asn Ile Thr Val Ala Thr Val Pro Ser Trp Leu Lys
                405                 410                 415

Lys Pro Gln Asp Ser Gln Leu Glu Glu Gly Lys Pro Gly Tyr Leu Asp
            420                 425                 430

Cys Leu Thr Gln Ala Thr Pro Lys Pro Thr Val Val Trp Tyr Arg Asn
        435                 440                 445

Gln Met Leu Ile Ser Glu Asp Ser Arg Phe Glu Val Phe Lys Asn Gly
    450                 455                 460

Thr Leu Arg Ile Asn Ser Val Glu Val Tyr Asp Gly Thr Trp Tyr Arg
465                 470                 475                 480

Cys Met Ser Ser Thr Pro Ala Gly Ser Ile Glu Ala Gln Ala Arg Val
                485                 490                 495

Gln Val Leu Glu Lys Leu Lys Phe Thr Pro Pro Pro Gln Pro Gln Gln
            500                 505                 510

Cys Met Glu Phe Asp Lys Glu Ala Thr Val Pro Cys Ser Ala Thr Gly

515                 520                 525

Arg Glu Lys Pro Thr Ile Lys Trp Glu Arg Ala Asp Gly Ser Ser Leu
    530                 535                 540

Pro Glu Trp Val Thr Asp Asn Ala Gly Thr Leu His Phe Ala Arg Val
545                 550                 555                 560

Thr Arg Asp Asp Ala Gly Asn Tyr Thr Cys Ile Ala Ser Asn Gly Pro
                565                 570                 575

Gln Gly Gln Ile Arg Ala His Val Gln Leu Thr Val Ala Val Phe Ile
            580                 585                 590

Thr Phe Lys Val Glu Pro Glu Arg Thr Thr Val Tyr Gln Gly His Thr
        595                 600                 605

Ala Leu Leu Gln Cys Glu Ala Gln Gly Asp Pro Lys Pro Leu Ile Gln
    610                 615                 620

Trp Lys Gly Lys Asp Arg Ile Leu Asp Pro Thr Lys Leu Gly Pro Arg
625                 630                 635                 640

Met His Ile Phe Gln Asn Gly Ser Leu Val Ile His Asp Val Ala Pro
                645                 650                 655

Glu Asp Ser Gly Arg Tyr Thr Cys Ile Ala Gly Asn Ser Cys Asn Ile
            660                 665                 670

Lys His Thr Glu Ala Pro Leu Tyr Val Val Asp Lys Pro Val Pro Glu
        675                 680                 685

Glu Ser Glu Gly Pro Gly Ser Pro Pro Pro Tyr Lys Met Ile Gln Thr
    690                 695                 700

Ile Gly Leu Ser Val Gly Ala Ala Val Ala Tyr Ile Ile Ala Val Leu
705                 710                 715                 720

Gly Leu Met Phe Tyr Cys Lys Lys Arg Cys Lys Ala Lys Arg Leu Gln
                725                 730                 735

<210>  8
<211>  23
<212>  DNA
<213>  Homo sapiens

<400>  8
ggaagccaca cttcacctag cag                                              23

<210>  9
<211>  23
<212>  DNA
<213>  Homo sapiens

<400> 9
ctgccacagt gagctggaca tgg 23

<210> 10
<211> 24
<212> DNA
<213> Homo sapiens

<400> 10
aagtgacaag accattgggg gagg 24

<210> 11
<211> 22
<212> DNA
<213> Homo sapiens

<400> 11
gggcatagtt attggcagca tc 22